# EUROPEAN PATENT APPLICATION

(11) **EP 4 668 287 A1**
(43) Date of publication of application: **24.12.2025**
(21) Application number: 25179149.7
(22) Date of filing: 27.05.2025
(51) Int. Cl.: G16H 40/40, G16H 30/40

(54) **SCANNER FAULT PREDICTION VIA IMAGE-BASED DEEP LEARNING**

(30) Priority: 21.06.2024 US 202418750327
(71) Applicant: GE Precision Healthcare LLC, Waukesha, WI 53188 (US)
(72) Inventor: RAMESH, Nithya, 560066 Bengaluru (IN)
(74) Representative: Kilburn & Strode LLP

(57) **Abstract**

Systems/techniques that facilitate scanner fault prediction via image-based deep learning are provided. In various embodiments, a system can access a medical image (e.g., via 106) captured by a medical imaging scanner (e.g., 104). In various aspects, the system can generate, via execution of at least one of one or more deep learning neural networks (e.g., 302) on the medical image, a failure classification label (e.g., 304) that indicates that the medical imaging scanner is afflicted by a first defined scanning failure from a plurality of defined scanning failures (e.g., 402). In various instances, the system can transmit an electronic notification (e.g., 1002) to a computing device associated with a technician of the medical imaging scanner, wherein the electronic notification can request that the medical imaging scanner be serviced to remedy the first defined scanning failure.

## Description

### TECHNICAL FIELD

The subject disclosure relates generally to medical imaging scanners, and more specifically to scanner fault prediction via image-based deep learning.

### BACKGROUND

A medical imaging scanner can capture or generate medical images of medical patients. Such medical images can be negatively affected by failures or malfunctions of the medical imaging scanner. To prevent such negative effects, such failures or malfunctions should be forecasted in advance. Existing techniques facilitate such forecasting by relying upon internal operability sensors of the medical imaging scanner. Unfortunately, many deployed medical imaging scanners are legacy devices that lack, and cannot be readily outfitted with, such internal operability sensors.

Accordingly, systems or techniques that can address one or more of these technical problems can be desirable.

### SUMMARY

The following presents a summary to provide a basic understanding of one or more embodiments. This summary is not intended to identify key or critical elements, or delineate any scope of the particular embodiments or any scope of the claims. Its sole purpose is to present concepts in a simplified form as a prelude to the more detailed description that is presented later. In one or more embodiments described herein, devices, systems, computer-implemented methods, apparatus or computer program products that facilitate scanner fault prediction via image-based deep learning are described.

According to one or more embodiments, a system is provided. The system can comprise a non-transitory computer-readable memory that can store computer-executable components. The system can further comprise a processor that can be operably coupled to the non-transitory computer-readable memory and that can execute the computer-executable components stored in the non-transitory computer-readable memory. In various embodiments, the computer-executable components can comprise an access component that can access a medical image captured by a medical imaging scanner. In various aspects, the computer-executable components can comprise a failure component that can generate, via execution of at least one of one or more deep learning neural networks on the medical image, a failure classification label that indicates that the medical imaging scanner is afflicted by a first defined scanning failure from a plurality of defined scanning failures. In various instances, the computer-executable components can comprise an action component that can transmit an electronic notification to a computing device associated with a technician of the medical imaging scanner, wherein the electronic notification requests that the medical imaging scanner be serviced to remedy the first defined scanning failure.

According to one or more embodiments, a computer-implemented method is provided. In various embodiments, the computer-implemented method can comprise accessing, by a device operatively coupled to a processor, a medical image captured by a medical imaging scanner. In various aspects, the computer-implemented method can comprise generating, by the device and via execution of at least one of one or more deep learning neural networks on the medical image, a failure classification label that indicates that the medical imaging scanner is afflicted by a first defined scanning failure from a plurality of defined scanning failures. In various instances, the computer-implemented method can comprise transmitting, by the device, an electronic notification to a computing device associated with a technician of the medical imaging scanner, wherein the electronic notification requests that the medical imaging scanner be serviced to remedy the first defined scanning failure.

According to one or more embodiments, a computer program product for facilitating scanner fault prediction via image-based deep learning is provided. In various embodiments, the computer program product can comprise a non-transitory computer-readable memory having program instructions embodied therewith. In various aspects, the program instructions can be executable by a processor to cause the processor to access a medical image captured by a medical imaging scanner. In various instances, the program instructions can be further executable to cause the processor to generate, via execution of at least one of one or more deep learning neural networks on the medical image, a failure classification label that indicates that the medical imaging scanner is afflicted by a first defined scanning failure from a plurality of defined scanning failures. In various cases, the program instructions can be further executable to cause the processor to transmit an electronic notification to a computing device associated with a technician of the medical imaging scanner, wherein the electronic notification requests that the medical imaging scanner be serviced to remedy the first defined scanning failure.

### DESCRIPTION OF THE DRAWINGS

FIG. 1 illustrates a block diagram of an example, non-limiting system that facilitates scanner fault prediction via image-based deep learning in accordance with one or more embodiments described herein.
FIG. 2 illustrates an example, non-limiting block diagram showing a medical imaging scanner and a digital twin in accordance with one or more embodiments described herein.
FIG. 3 illustrates a block diagram of an example, non-limiting system including a first deep learning neural network and a failure classification label that facilitates scanner fault prediction via image-based deep learning in accordance with one or more embodiments described herein.
FIG. 4 illustrates an example, non-limiting block diagram showing how a first deep learning neural network can generate a failure classification label in accordance with one or more embodiments described herein.
FIG. 5 illustrates a block diagram of an example, non-limiting system including a second deep learning neural network and a root cause classification label that facilitates scanner fault prediction via image-based deep learning in accordance with one or more embodiments described herein.
FIG. 6 illustrates an example, non-limiting block diagram showing how a second deep learning neural network can generate a root cause classification label in accordance with one or more embodiments described herein.
FIG. 7 illustrates a block diagram of an example, non-limiting system including a third deep learning neural network and a remaining useful life that facilitates scanner fault prediction via image-based deep learning in accordance with one or more embodiments described herein.
FIG. 8 illustrates an example, non-limiting block diagram showing how a third deep learning neural network can estimate a remaining useful life in accordance with one or more embodiments described herein.
FIG. 9 illustrates an example, non-limiting block diagram showing a cascaded deep learning pipeline for predicting medical imaging scanner faults in accordance with one or more embodiments described herein.
FIG. 10 illustrates a block diagram of an example, non-limiting system including a service request that facilitates scanner fault prediction via image-based deep learning in accordance with one or more embodiments described herein.
FIG. 11 illustrates an example, non-limiting block diagram showing a service request in accordance with one or more embodiments described herein.
FIGs. 12-14 illustrate example, non-limiting block diagrams showing how a service tutorial can be generated in accordance with one or more embodiments described herein.
FIG. 15 illustrates a block diagram of an example, non-limiting system including a digital twin synchronization that facilitates scanner fault prediction via image-based deep learning in accordance with one or more embodiments described herein.
FIG. 16 illustrates an example, non-limiting block diagram showing how a digital twin can be synchronized to a medical imaging scanner in accordance with one or more embodiments described herein.
FIG. 17 illustrates an example, non-limiting block diagram showing how artificial intelligence models can be trained in accordance with one or more embodiments described herein.
FIG. 18 illustrates a flow diagram of an example, non-limiting computer-implemented method that facilitates scanner fault prediction via image-based deep learning in accordance with one or more embodiments described herein.
FIG. 19 illustrates a block diagram of an example, non-limiting operating environment in which one or more embodiments described herein can be facilitated.
FIG. 20 illustrates an example networking environment operable to execute various implementations described herein.

### DETAILED DESCRIPTION

The following detailed description is merely illustrative and is not intended to limit embodiments or application/uses of embodiments. Furthermore, there is no intention to be bound by any expressed or implied information presented in the preceding Background or Summary sections, or in the Detailed Description section.

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

A medical imaging scanner (e.g., a computed tomography (CT) scanner, a magnetic resonance imaging (MRI) scanner, an X-ray scanner, an ultrasound scanner, a positron emission tomography (PET) scanner, a nuclear medicine (NM) scanner) can capture or generate medical images (e.g., scanned CT images, scanned MRI images, scanned X-ray images, scanned ultrasound images, scanned PET images, scanned NM images) of medical patients (e.g., humans, animals, or otherwise). Such medical images can be negatively affected by failures or malfunctions of the medical imaging scanner. For instance, when one or more constituent hardware components (e.g., X-ray tube, X-ray detector, anode, cathode, focus coil, deflection coil, gantry motor, table motor) of the medical imaging scanner break, such breakage can cause medical images captured by the medical imaging scanner to develop imaging artifacts (e.g., phantom streaks, phantom shadows, phantom rings, phantom contours) or other visual quality issues (e.g., blurriness, visual noise, optical distortions). In other words, hardware failures or malfunctions of the medical imaging scanner can cause degradation of the medical images captured or generated by the medical imaging scanner. When such hardware failures or malfunctions are nascent or otherwise just beginning (e.g., when a hardware component is just starting to fail but has not yet fully or severely failed), such medical image degradation can be slight (e.g., possibly not noticeable to the naked eye). At such point, servicing of the medical imaging scanner can be warranted but not yet critical. However, when such hardware failures or malfunctions become mature (e.g., when a hardware component has fully or otherwise severely failed), such medical image degradation can be significant (e.g., conspicuously corrupting image content). At such point, servicing of the medical imaging scanner can be critically necessary.

To prevent such negative imaging outcomes, and to otherwise reduce or avoid unplanned downtime of the medical imaging scanner, it can be desired to forecast the occurrence of failures or malfunctions of the medical imaging scanner in advance. Existing techniques facilitate such forecasting by relying upon internal operability sensors of the medical imaging scanner. For instance, such existing techniques involve outfitting each given hardware component of the medical imaging scanner with various voltage sensors, current sensors, resistors, thermistors, pressure sensors, potentiometers, transistors, or other electronic sensors, where such sensors monitor the operational state (e.g., the incoming or outgoing voltages, the incoming or outgoing currents, the internal temperatures, the incoming or outgoing contact forces) of that given hardware component over time. Any point in time at which the readouts of those sensors satisfy any suitable threshold values can be considered a point in time at which the given hardware component is functioning appropriately or otherwise as expected. In contrast, any point in time at which the readouts of those sensors do not satisfy any suitable threshold values can be considered a point in time at which the given hardware component is not functioning appropriately or otherwise as expected. Thus, the internal operability sensors of the medical imaging scanner can be considered as providing time-series readouts that allow identification of hardware failures or malfunctions.

Unfortunately, many deployed medical imaging scanners are legacy devices (e.g., scanners that are years or decades old) that lack, and cannot be readily retrofitted with, such internal operability sensors. Thus, existing techniques cannot be applied to such medical imaging scanners.

Accordingly, systems or techniques that can address one or more of these technical problems can be desirable.

Various embodiments described herein can address one or more of these technical problems. One or more embodiments described herein can include systems, computer-implemented methods, apparatus, or computer program products that can facilitate scanner fault prediction via image-based deep learning. In particular, the inventors of various embodiments described herein realized that different types or severities of medical imaging scanner failures or malfunctions can result in the manifestation of different types or severities of image degradation. In other words, the present inventors recognized that respective scanner failures or malfunctions can be considered as leaving unique fingerprints on captured or generated medical images, and the present inventors further recognized that artificial intelligence models (e.g., deep learning neural networks) can be trained or otherwise configured to detect such unique fingerprints. Accordingly, various embodiments described herein can execute deep learning neural networks on captured or generated medical images, so as to forecast hardware failures of medical imaging scanners. More specifically, various embodiments described herein can involve a cascaded deep learning pipeline that includes three distinct deep learning neural networks. When given a medical image captured by a medical imaging scanner, a first deep learning neural network in the cascaded pipeline can be executed on the medical image, thereby yielding a first classification label that indicates a type of scanner failure that the medical imaging scanner is experiencing (e.g., the medical image can contain unique visual features or attributes, and the first deep learning neural network can be trained to map those unique visual features or attributes to a specific scanner failure). In various aspects, a second deep learning neural network in the cascaded pipeline can be executed on both the medical image and the first classification label, thereby yielding a second classification label that indicates which hardware component of the medical imaging scanner is the root cause of whatever failure is indicated in the first classification label (e.g., the medical image can contain unique visual features or attributes, and the second deep learning neural network can, when conditioned on a specific scanner failure, be trained to map those unique visual features or attributes to the malfunction of a specific hardware component of the medical imaging scanner). **In** various instances, a third deep learning neural network in the cascaded pipeline can be executed on all of the medical image, the first classification label, and the second classification label, thereby yielding a remaining useful life of whatever hardware component is indicated by the second classification label (e.g., the medical image can contain unique visual features or attributes, and the third deep learning neural network can, when conditioned on a specific scanner failure and a specific hardware component, be trained to map those unique visual features or attributes to how many operating hours that specific hardware component has left). Note that each deep learning neural network in the cascaded pipeline can receive as input the medical image and the outputs of the deep learning neural networks that come before it, hence the term "cascaded". However, such cascaded pipeline is a mere non-limiting example. In other cases, various embodiments described herein can implement any other suitable arrangement or layout of deep learning neural networks so as to predict failure mode, root cause of failure mode, or remaining useful life of the medical imaging scanner, based on images captured by the medical imaging scanner rather than based on time-series readouts of internal operability sensors of the medical imaging scanner (e.g., in some situations, the first, second, and third deep learning neural networks can be not cascaded, such that no model receives as input the output of another model; in other situations, a single model can simultaneously predict failure mode, root cause, and remaining useful life based on an inputted medical image). In any case, various embodiments described herein can be considered as a clever utilization of artificial intelligence models that enable detection or prediction of faults or failures of medical imaging scanners based on the images captured or generated by such medical imaging scanners, even if such medical imaging scanners lack or exclude internal operability sensors.

Various embodiments described herein can be considered as a computerized tool (e.g., any suitable combination of computer-executable hardware or computer-executable software) that can facilitate scanner fault prediction via image-based deep learning. In various aspects, such computerized tool can comprise an access component, a failure component, a cause component, a life component, or an action component.

In various embodiments, there can be a medical imaging scanner. In various aspects, the medical imaging scanner can be any suitable medical modality, equipment, or device that can capture or generate medical scanned images (e.g., CT scanner, X-ray scanner, MRI scanner). In various instances, the medical imaging scanner can be considered as being made up or otherwise constructed of a plurality of hardware components (e.g., X-ray tubes, X-ray detectors, gantry motors, table motors).

In various embodiments, the medical imaging scanner can capture or generate a medical image. In various aspects, the medical image can exhibit any suitable format, size, or dimensionality (e.g., the medical image can be a two-dimensional pixel array, or the medical image can be a three-dimensional voxel array). In various instances, the medical image can visually depict any suitable anatomical structure (e.g., tissue, organ, body part, or portion thereof) of any suitable medical patient.

In various cases, the medical imaging scanner can lack or otherwise exclude internal operability sensors, such that the specific operating states of the plurality of hardware components cannot be monitored in real-time. Nevertheless, it can be desired to detect whether or not the medical imaging scanner or any constituent hardware components thereof are experiencing failures or malfunctions. As described herein, the computerized tool can facilitate such detection.

In various embodiments, the access component of the computerized tool can electronically access the medical imaging scanner. For instance, the access component can electronically interface or communicate with (e.g., send electronic commands to, read electronic signals from) the medical imaging scanner. Furthermore, in various aspects, the access component can electronically access the medical image. That is, the access component can receive, retrieve, or obtain the medical image from any suitable centralized or decentralized data structures (e.g., graph data structures, relational data structures, hybrid data structures), whether remote from or local to the access component (e.g., can obtain the medical image from the medical imaging scanner itself). In any case, the access component can be considered as a conduit through which other components of the computerized tool can electronically interact with (e.g., read, write, edit, copy, manipulate, execute, activate, deactivate, modify) the medical imaging scanner or the medical image.

In various embodiments, the failure component of the computerized tool can electronically store, maintain, control, or otherwise access a first deep learning neural network. In various aspects, the first deep learning neural network can exhibit any suitable deep learning internal architecture. For example, the first deep learning neural network can include any suitable numbers of any suitable types of layers (e.g., input layer, one or more hidden layers, output layer, any of which can be convolutional layers, dense layers, long short-term memory (LSTM) layers, non-linearity layers, pooling layers, batch normalization layers, or padding layers). As another example, the first deep learning neural network can include any suitable numbers of neurons in various layers (e.g., different layers can have the same or different numbers of neurons as each other). As yet another example, the first deep learning neural network can include any suitable activation functions (e.g., softmax, sigmoid, hyperbolic tangent, rectified linear unit) in various neurons (e.g., different neurons can have the same or different activation functions as each other). As still another example, the first deep learning neural network can include any suitable interneuron connections or interlayer connections (e.g., forward connections, skip connections, recurrent connections).

Regardless of its specific internal architecture, the first deep learning neural network can be configured as an image classifier. That is, the first deep learning neural network can be configured to receive as input any image and to determine as output to which of a plurality of defined classes the inputted image belongs. Accordingly, the failure component can electronically execute the first deep learning neural network on the medical image, and such execution can yield a first classification label. More specifically, the failure component can feed the medical image to the input layer of the first deep learning neural network, the medical image can complete a forward pass through the one or more hidden layers of the first deep learning neural network, and the output layer of the first deep learning neural network can calculate the first classification label based on activations provided by the one or more hidden layers of the first deep learning neural network.

In various aspects, the first classification label can indicate a failure mode that the first deep learning neural network believes or infers that the medical imaging scanner is experiencing. In particular, there can be a plurality of defined failure modes that the medical imaging scanner can possibly experience (e.g., a tube arcing failure mode, a misalignment failure mode, a condensation obfuscation failure mode), and the first classification label can indicate which one of the plurality of defined failure modes that the medical imaging scanner is (in the opinion of the first deep learning neural network) currently or presently afflicted by. In other words, the medical image can be considered as containing unique or distinctive visual details (e.g., artifacts, patterns, blurriness, noise, distortions, any of which might not even be noticeable to the naked eye), and the first deep learning neural network can be considered as inferring which of the plurality of defined failure modes is correlated, mapped, or otherwise causally linked to such unique or distinctive visual details. Note that, in some cases, the first classification label can include a "no failure mode detected" class, so as to address situations in which the medical imaging scanner is operating healthily (e.g., situations in which the medical image has no malfunction-indicative artifacts, patterns, blurriness, noise, or distortions).

In various embodiments, the cause component of the computerized tool can electronically store, maintain, control, or otherwise access a second deep learning neural network. In various aspects, the second deep learning neural network can exhibit any suitable deep learning internal architecture. For example, the second deep learning neural network can include any suitable numbers of any suitable types of layers (e.g., input layer, one or more hidden layers, output layer, any of which can be convolutional layers, dense layers, LSTM layers, non-linearity layers, pooling layers, batch normalization layers, or padding layers). As another example, the second deep learning neural network can include any suitable numbers of neurons in various layers (e.g., different layers can have the same or different numbers of neurons as each other). As yet another example, the second deep learning neural network can include any suitable activation functions (e.g., softmax, sigmoid, hyperbolic tangent, rectified linear unit) in various neurons (e.g., different neurons can have the same or different activation functions as each other). As still another example, the second deep learning neural network can include any suitable interneuron connections or interlayer connections (e.g., forward connections, skip connections, recurrent connections).

Regardless of its specific internal architecture, the second deep learning neural network can be configured as an image classifier that is conditioned on the output of the first deep learning neural network. That is, the second deep learning neural network can be configured to receive as input any image and whatever classification label that the first deep learning neural network has produced for that inputted image, and to determine as output to which of another plurality of defined classes the inputted image belongs. Accordingly, in response to the first classification label indicating something other than the "no failure mode detected" class, the cause component can electronically execute the second deep learning neural network on the medical image and on the first classification label, and such execution can yield a second classification label. More specifically, the cause component can concatenate the medical image and the first classification label together, the cause component can feed that concatenation to the input layer of the second deep learning neural network, that concatenation can complete a forward pass through the one or more hidden layers of the second deep learning neural network, and the output layer of the second deep learning neural network can calculate the second classification label based on activations provided by the one or more hidden layers of the second deep learning neural network.

In various aspects, the second classification label can indicate a root cause that the second deep learning neural network believes or infers is responsible for whatever defined failure mode is indicated in the first classification label. In particular, as mentioned above, the medical imaging scanner can be made up of a plurality of hardware components. In various instances, any given failure mode can have multiple possible causes. That is, the given failure mode can, in some cases, be caused by some particular hardware component being damaged or worn (e.g., a tube arcing failure can be caused by a degraded cathode), but the given failure can, in other cases, be caused by some different hardware component being damaged or worn (e.g., a tube arcing failure can alternatively be caused by a damaged vacuum seal or gasket). In various cases, the second classification label can indicate which one of the plurality of hardware components is (in the opinion of the second deep learning neural network) currently or presently malfunctioning and thus responsible for the failure mode that is indicated by the first classification label. Indeed, the medical image can be considered as containing unique or distinctive visual details, and the second deep learning neural network can be considered as inferring which of the plurality of hardware components is correlated, mapped, or otherwise causally linked to such unique or distinctive visual details, given that such unique or distinctive visual details have already been mapped to a respective failure mode by the first deep learning neural network. In other words, the output of the first deep learning neural network can be considered as supplemental input information that assists the second deep learning neural network in generating its own output.

In various embodiments, the life component of the computerized tool can electronically store, maintain, control, or otherwise access a third deep learning neural network. In various aspects, the third deep learning neural network can exhibit any suitable deep learning internal architecture. For example, the third deep learning neural network can include any suitable numbers of any suitable types of layers (e.g., input layer, one or more hidden layers, output layer, any of which can be convolutional layers, dense layers, LSTM layers, non-linearity layers, pooling layers, batch normalization layers, or padding layers). As another example, the third deep learning neural network can include any suitable numbers of neurons in various layers (e.g., different layers can have the same or different numbers of neurons as each other). As yet another example, the third deep learning neural network can include any suitable activation functions (e.g., softmax, sigmoid, hyperbolic tangent, rectified linear unit) in various neurons (e.g., different neurons can have the same or different activation functions as each other). As still another example, the third deep learning neural network can include any suitable interneuron connections or interlayer connections (e.g., forward connections, skip connections, recurrent connections).

Regardless of its specific internal architecture, the third deep learning neural network can be configured as an image regressor that is conditioned on the outputs of the first and second deep learning neural networks. That is, the third deep learning neural network can be configured to receive as input any image and whatever classification labels that the first and second deep learning neural networks have produced for that inputted image, and to produce as output a continuously-variable quantity for that inputted image. Accordingly, the life component can electronically execute the third deep learning neural network on the medical image, on the first classification label, and on the second classification label, and such execution can yield a remaining useful life (RUL) value. More specifically, the life component can concatenate the medical image, the first classification label, and the second classification label together, the life component can feed that concatenation to the input layer of the third deep learning neural network, that concatenation can complete a forward pass through the one or more hidden layers of the third deep learning neural network, and the output layer of the third deep learning neural network can calculate the RUL value based on activations provided by the one or more hidden layers of the third deep learning neural network.

In various aspects, the RUL value can be a real-valued scalar that indicates how much useful life (e.g., in terms of operating hours) that whichever hardware component indicated by the second classification label has remaining. In particular, the first classification label can indicate a specific failure mode of the medical imaging scanner, and the second classification label can indicate a specific hardware component of the medical imaging scanner that is malfunctioning and thereby causing the specific failure mode. In various cases, the severity of the specific failure mode, and thus the noticeability of its visual manifestations in the medical image, can vary across a range or spectrum commensurately with a severity of malfunctioning of the specific hardware component. For instance, if the specific hardware component is only slightly malfunctioning, then the specific failure mode can have a commensurately slight severity, which can result in commensurately slight visual degradation of (e.g., barely-noticeable artifacts or blurriness in) the medical image. However, if the specific hardware component is instead significantly malfunctioning, then the specific failure mode can have a commensurately significant severity, which can result in commensurately significant visual degradation of (e.g., noticeably apparent artifacts or blurriness in) the medical image. Furthermore, the severity of the specific failure mode can vary inversely with the remaining useful life of the specific hardware component. Indeed, if the specific hardware component is only slightly malfunctioning, then the specific hardware component can have a sizeable or lengthy amount of time remaining in which it can be usefully operated. However, if the specific hardware component is instead significantly malfunctioning, then the specific hardware component can have a small or short amount of time remaining in which it can be usefully operated. So, the medical image can be considered as containing unique or distinctive visual details, and the third deep learning neural network can be considered as inferring how much remaining useful life is correlated, mapped, or otherwise linked to such unique or distinctive visual details, given that such unique or distinctive visual details have already been mapped to the specific failure mode and to the specific hardware component by the first and second deep learning neural networks respectively. In other words, the outputs of the first and second deep learning neural networks can be considered as supplemental input information that assists the third deep learning neural network in generating its own output.

In various embodiments, the action component of the computerized tool can initiate or perform any suitable electronic actions based on the outputs of the first, second, and third deep learning neural networks. For instance, in response to the first classification label indicating anything other than the "no failure mode detected" class, the action component can electronically transmit a service request to any suitable computing device associated with a technician that uses or oversees the medical imaging scanner. In such cases, the service request can be any suitable electronic message that includes or contains the first classification label, the second classification label, or the RUL value. In other words, the service request can be considered as instructing or commanding the technician to make a service visit to the medical imaging scanner, where such service visit will involve fixing whatever failure mode is indicated by the first classification label by repairing or replacing whatever hardware component is indicated by the second classification label, and where such service visit is required before the RUL value elapses. Note that the action component can be considered not just as automatically scheduling maintenance for the medical imaging scanner, but also as proactively providing to the technician information (e.g., the first and second classification labels and the RUL value) that will aid the technician in expediting such maintenance.

In other cases, as described herein, the action component can synthesize (e.g., via retrievable-augmented execution of a large language model (LLM), such as ChatGPT) a textual tutorial that helps to teach or explain to the technician how to repair or replace whichever hardware component is indicated by the second classification label.

In yet other cases, as described herein, the action component can update or synchronize a digital twin with the medical imaging scanner, by leveraging the first and second classification labels and the RUL value.

In any case, the computerized tool described herein can be considered as automatically detecting, predicting, or otherwise forecasting failures or malfunctions of the medical imaging scanner, even in the absence of internal operability sensors that monitor the plurality of hardware components that make up the medical imaging scanner. As described herein, the computerized tool can facilitate such automated detection, prediction, or forecasting by leveraging a cascaded deep learning pipeline in which each neural network receives as input a common medical image produced by the medical imaging scanner and whatever outputs are produced by upstream neural networks in the pipeline. However, such cascaded pipeline is a mere non-limiting example. In other cases, any other suitable deep learning pipeline can be implemented so as to perform failure classification, root cause classification, or RUL estimation on a given medical image.

As a non-limiting example, the first, second, and third deep learning neural networks can be arranged in a serial, non-cascaded layout, such that no network receives as input the output of another network. In such case, the second deep learning neural network can receive as input only the medical image (e.g., cannot receive as input the failure classification label). Likewise, in such case, the third deep learning neural network can receive as input only the medical image (e.g., can receive as input neither the failure classification label nor the root cause classification label). In such situations, the second deep learning neural network can be executed only when the failure classification label indicates something other than the "no failure mode detected" class. Moreover, in such situations, there can be a plurality of third deep learning neural networks, each one being configured or trained to predict the remaining useful life of a respective hardware component of the medical imaging scanner (e.g., one network trained or configured to estimate remaining useful life of an X-ray tube anode; another network trained or configured to estimate remaining useful life of an X-ray tube cathode). Accordingly, whichever of that plurality of third deep learning neural networks corresponds to whichever hardware component is indicated by the root cause classification label can be executed on the medical image to generate the RUL estimation.

As another non-limiting example, the second deep learning neural network can be excluded or omitted, and the first and third deep learning neural networks can be arranged in a serial, non-cascaded layout. In such case, there can be no root cause classification label. Instead, the first deep learning neural network can receive as input the medical image and can produce as output the failure classification label; and, if the failure classification label indicates something other than the "no failure mode detected" class, the third deep learning neural network can then be executed on the medical image (e.g., not receiving the failure classification label as input) to produce as output the RUL estimation. In such situations, the RUL estimation can be considered as indicating a remaining useful life for the medical imaging scanner as a whole, rather than for any specific or individual hardware component of the medical imaging scanner. Such embodiments can nevertheless be considered as useful from a technician's perspective, since whatever failure mode is indicated by the failure classification label can be considered as conveying to the technician which general or coarse subset of hardware components of the medical imaging scanner should be investigated, repaired, or replaced (e.g., tube arcing failure mode indicates that X-ray tube components should be investigated, repaired, or replaced; condensation obfuscation failure mode indicates that detector or optical lenses should be investigated, repaired, or replaced).

As even another non-limiting example, the first deep learning neural network can be excluded or omitted, and the second and third deep learning neural networks can be arranged in a serial, non-cascaded layout. In such case, there can be no failure classification label. Instead, the second deep learning neural network can receive as input the medical image alone and can produce as output the root cause classification label as described above, where the root cause classification label can have an additional "no hardware component is malfunctioning" class. Accordingly, if the root cause classification label indicates something other than the "no hardware component is malfunctioning" class, the third deep learning neural network can then be executed on the medical image (e.g., not receiving the root cause classification label as input) to produce as output the RUL estimation. In some of such situations, the RUL estimation can be considered as indicating a remaining useful life for the medical imaging scanner as a whole, rather than for any specific or individual hardware component of the medical imaging scanner. However, in other of such situations, there can (as mentioned above) be a plurality of third deep learning neural networks each configured to estimate remaining useful life of a respective hardware component, and whichever of that plurality of third deep learning neural networks corresponds to the specific hardware component indicated by the root cause classification label can be executed on the medical image alone to produce the RUL estimation.

As still another non-limiting example, the first, second, and third deep learning neural networks can be arranged in a serial, non-cascaded layout, where the third deep learning neural network can be executed before the first and second deep learning neural networks. In such case, the third deep learning neural network can receive as input the medical image alone and can produce as output an RUL estimation for the medical imaging scanner as a whole. If the RUL estimation is below any suitable threshold, it can be concluded or expected that something is wrong with the medical imaging scanner. In response to such conclusion or expectation, the first or second deep learning neural networks can then be executed on the medical image alone, thereby identifying what specific failure mode or what specific hardware component malfunction the medical imaging scanner is experiencing.

As yet another non-limiting example, the first and second deep learning neural networks can be excluded or omitted, and a plurality of third deep learning neural networks can be implemented in parallel with each other, where each of the plurality of third deep learning neural networks is configured or trained to estimate remaining useful life of a respective hardware component of the medical imaging scanner. In such case, each of the plurality of third deep learning neural networks can be independently executed on the medical image, thereby yielding a plurality of RUL estimations respectively corresponding to all the hardware components that make up the medical imaging scanner. If any given RUL estimation of that plurality of RUL estimations fails to satisfy any suitable threshold, it can be concluded that whichever hardware component corresponds to that given RUL estimation is malfunctioning or otherwise in need of investigation, repair, or replacement.

No matter what specific deep learning pipeline is implemented, evidence of a malfunction of the medical imaging scanner can be visually manifested in images captured by the medical imaging scanner, and various embodiments described herein can utilize deep learning to detect or otherwise recognize such visually-manifested evidence. In this way, malfunctions or failures of the medical imaging scanner can be proactively predicted or dealt with, even if the medical imaging scanner lacks internal operability sensors.

Note that, in order for detection, prediction, or forecasting to be accurately or correctly performed, the herein-described machine learning models (e.g., the first, second, and third deep learning neural networks) should first undergo training. In various cases, the computerized tool can train such machine learning models using any suitable training paradigms (e.g., via supervised training, unsupervised training, or reinforcement learning).

Various embodiments described herein can be employed to use hardware or software to solve problems that are highly technical in nature (e.g., to facilitate scanner fault prediction via image-based deep learning), that are not abstract and that cannot be performed as a set of mental acts by a human. Further, some of the processes performed can be performed by a specialized computer (e.g., image classifiers, image regressors, LLMs, digital twins) for carrying out defined acts related to medical imaging scanners.

For example, such defined acts can include: accessing, by a device operatively coupled to a processor, a medical image captured by a medical imaging scanner; generating, by the device and via execution of at least one of one or more deep learning neural networks on the medical image, a failure classification label that indicates that the medical imaging scanner is afflicted by a first defined scanning failure from a plurality of defined scanning failures; and transmitting, by the device, an electronic notification to a computing device associated with a technician of the medical imaging scanner, wherein the electronic notification requests that the medical imaging scanner be serviced to remedy the first defined scanning failure.

In various instances, such defined acts can include: generating, by the device and via execution of at least one of the one or more deep learning neural networks on the medical image and on the failure classification label, a root cause classification label that indicates that a first defined hardware component, from a plurality of defined hardware components that make up the medical imaging scanner, is malfunctioning and thereby causing the first defined scanning failure, wherein the electronic notification indicates that the first defined scanning failure is curable by repairing or replacing the first defined hardware component.

In various cases, such defined acts can include: estimating, by the device and via execution of at least one of the one or more deep learning neural networks on the medical image, on the failure classification label, and on the root cause classification label, a first remaining useful life for the first defined hardware component, wherein the electronic notification includes the first remaining useful life.

In various aspects, such defined acts can include synthesizing, by the device and via execution of a large language model, a textual tutorial explaining how to repair or replace the first defined hardware component so as to remedy the first defined scanning failure, wherein the electronic notification can include the textual tutorial.

In various instances, such defined acts can include, in response to a determination that the first remaining useful life does not match a second remaining useful life estimated by a digital twin of the medical imaging scanner, updating or synchronizing the digital twin, based on the failure classification label, on the root cause classification label, and on the first remaining useful life.

Such defined acts are inherently computerized. Indeed, medical imaging scanners (e.g., CT scanners, MRI scanners, X-ray scanners) are computerized systems that capture scanned images of medical patients by leveraging specific clinical hardware (e.g., X-ray tubes, X-ray detectors, gantries). A medical imaging scanner cannot be implemented in any way whatsoever by the human mind or by humans with mere pen and paper. Likewise, neither the human mind nor a human with mere pen and paper can generate or capture scanned medical images (e.g., X-ray scanned images, CT scanned images) of medical patients. Moreover, artificial neural networks (e.g., image classifiers, image regressors, LLMs) are also inherently computerized constructs comprising specific software-oriented architectures (e.g., input layers, hidden layers, or output layers, any of which can be made up of trainable or non-trainable internal parameters such as convolutional layers or LSTM layers). Artificial neural networks cannot be trained or executed by the human mind, or by humans with mere pen and paper, in any reasonable or practicable way without computers. Furthermore, a digital twin (as the word "digital" in its name suggests) is also an inherently computerized or virtual construct that is used to electronically forecast or simulate future behavior of medical imaging scanners. A digital twin simply cannot be facilitated or executed by the human mind, or by a human with mere pen and paper, in any reasonable or practicable way without computers.

Moreover, various embodiments described herein can integrate into a practical application various teachings relating to scanner fault prediction via image-based deep learning. As described above, failures or malfunctions of a medical imaging scanner can cause the medical images that are captured by that medical imaging scanner to exhibit visual degradation (e.g., to have visual artifacts, blurriness, or otherwise optical distortions). To avoid such visual degradation, it can be desired to forecast the occurrence of such failures or malfunctions. Existing techniques facilitate such forecasting by analyzing (e.g., via threshold comparison) time-series readouts measured by internal operability sensors (e.g., voltage sensors, current sensors, resistors, thermistors, potentiometers, strain gauges) that monitor respective hardware components of the medical imaging scanner. Unfortunately, many legacy medical imaging scanners are not outfitted with such internal operability sensors, and retrofitting those legacy devices with such internal operability sensors can be prohibitively expensive.

Various embodiments described herein can ameliorate such technical problems, by leveraging image-based deep learning. That is, various embodiments described herein utilize the pattern recognition capabilities of deep learning neural networks to detect, within scanned medical images, the unique or distinct visual indications, signatures, or manifestations of respective scanner failures or malfunctions. Thus, failures or malfunctions can be forecasted, even for medical imaging scanners that lack internal operability sensors. In particular, various embodiments described herein can involve a cascaded deep learning pipeline that is made up of three deep learning neural networks. A first deep learning neural network in such cascaded pipeline can receive as input a medical image captured by a medical imaging scanner and can produce as output a failure classification label indicating from which specific failure mode (e.g., tube arcing failure, misalignment failure, condensation failure) the medical imaging scanner suffers. A second deep learning neural network in such cascaded pipeline can receive as input the medical image and the failure classification label and can produce as output a root cause classification label indicating which specific hardware component (e.g., X-ray tube anode, X-ray tube cathode, X-ray tube vacuum seal) of the medical imaging scanner is causing the specific failure mode. A third deep learning neural network in such cascaded pipeline can receive as input the medical image, the failure classification label, and the root cause classification label and can estimate as output a remaining useful life (e.g., remaining hours of operation) that the specific hardware component has. Such pipeline can be referred to as "cascaded" since such three deep learning neural networks can be organized in series with each other such each deep learning neural network receives as input the medical image and the outputs of all previous deep learning neural networks.

In various aspects, such cascading can be considered as a clever, innovative, or otherwise beneficial structure or organization for facilitating image-based scanner fault prediction. Indeed, each neural network can be considered as adding an incremental amount of supplemental or enriching information that can improve the ability of the successive or next neural network to perform its own inferencing task. Stated differently, the cascaded deep learning pipeline can be considered as an artificial chain-of-thought that iteratively enriches the medical image and thereby allows for improved scanner fault prediction accuracy. Stated differently again, the cascaded deep learning pipeline can be considered as breaking the overall problem of scanner fault prediction up into discrete, more manageable mini-tasks (e.g., fault classification, followed by root cause classification, followed by remaining useful life regression) that each respective deep learning neural network can master. This can help to avoid problems such as overfitting or overlearning.

For example, the failure classification performed by the first deep learning neural network can be considered as supplemental context for the medical image, and such supplemental context can assist the second deep learning neural network in performing root cause classification. In other words, the second deep learning neural network could exhibit reduced root cause classification accuracy or generalizability if it were to receive as input just the medical image alone (e.g., the conclusion of the first deep learning neural network can help to direct or guide the second deep learning neural network). As another example, both the failure classification performed by the first deep learning neural network and the root cause classification performed by the second deep learning neural network can be considered as supplemental context for the medical image, and such supplemental context can assist the third deep learning neural network in performing remaining useful life regression. That is, the third deep learning neural network could exhibit reduced remaining useful life regression accuracy or generalizability if it were to receive as input just the medical image alone (e.g., the conclusions of the first and second deep learning neural networks can help to direct or guide the third deep learning neural network).

Moreover, such cascaded pipeline can be considered as exhibiting increased or otherwise heightened explainability or interpretability. Indeed, various embodiments described herein can involve training or otherwise configuring a single deep learning neural network to receive as input the medical image and to produce as output the failure classification label, the root cause classification label, and the remaining useful life all at the same time. However, such single deep learning neural network could be considered as a black-box that performs all three of failure classification, root cause classification, and remaining useful life regression on the medical image in an inexplicable or uninterpretable fashion that renders future fine-tuning or model auditing difficult (e.g., if any one of the failure classification, root cause classification, or remaining useful life regression is determined to have been inaccurately performed, then the entirety of the single deep learning neural network would have to be fine-tuned to address such inaccuracy). In contrast, sequentially performing failure classification, root cause classification, and remaining useful life regression in cascaded fashion can be considered as a more transparent, explainable, or interpretable proof-of-work technique (e.g., if any one of the failure classification, root cause classification, or remaining useful life regression is determined to have been inaccurately performed, then only the corresponding neural network in the cascaded pipeline can be fine-tuned to address such inaccuracy, and the other neural networks can be left as they are). Such heightened explainability or interpretability constitutes another advantage of various embodiments described herein. Indeed, explainable artificial intelligence is a burgeoning area of research and development which seeks to reduce the black-box opaqueness that normally accompanies deep learning.

However, as mentioned above, various embodiments described herein can implement any other suitable, non-cascaded deep learning layout, arrangement, or pipeline (e.g., three networks executed serially but without the output of any being fed as input to the others; a respective RUL estimation network being trained or configured for each defined hardware component of the medical imaging scanner; RUL estimation network being executed prior to failure or root cause classification networks). No matter what specific deep learning layout, arrangement, or pipeline is implemented, various embodiments described herein can execute deep learning models on scanned images produced by a medical imaging scanner, so as to infer malfunction information or failure information of the medical imaging scanner. Therefore, the medical imaging scanner can be proactively serviced or repaired, even in the absence of internal operability sensors (e.g., even if the medical imaging scanner is not outfitted with a myriad of internal sensors that provide a constant stream of time-series readout data explicitly indicating the medical imaging scanner's health or wear levels).

For at least these reasons, various embodiments described herein certainly constitute a tangible and concrete technical improvement or technical advantage in the field of medical imaging scanners. Accordingly, such embodiments clearly qualify as useful and practical applications of computers.

Furthermore, various embodiments described herein can control real-world tangible devices based on the disclosed teachings. For example, various embodiments described herein can execute artificial neural networks on real-world medical images captured by real-world medical imaging scanners (e.g., X-ray scanners, MRI scanners), and can schedule real-world servicing or maintenance for such real-world medical imaging scanners based on the outputs of those artificial neural networks.

It should be appreciated that the herein figures and description provide non-limiting examples of various embodiments and are not necessarily drawn to scale.

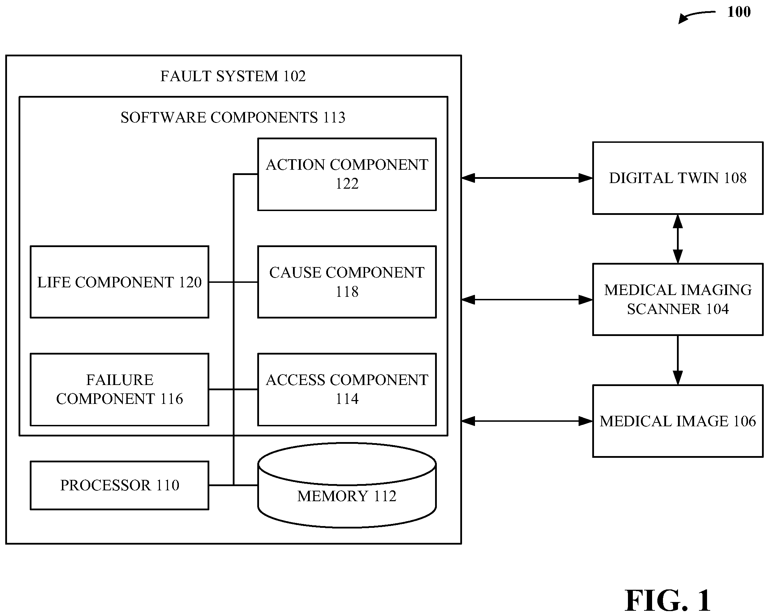

FIG. 1 illustrates a block diagram of an example, non-limiting system 100 that can facilitate scanner fault prediction via image-based deep learning in accordance with one or more embodiments described herein. As shown, a fault system 102 can be electronically integrated with a medical imaging scanner 104, with a medical image 106, or with a digital twin 108.

In various embodiments, the medical imaging scanner 104 can be any suitable medical image-capture modality or equipment that can capture or otherwise generate (e.g., via X-ray emission or electromagnetism) medical images. As a non-limiting example, the medical imaging scanner 104 can be an X-ray scanner that is configured to capture or generate X-ray scanned images of any suitable anatomical structures (e.g., organs, tissues, bodily cavities, bodily fluids) of any suitable medical patients. As another non-limiting example, the medical imaging scanner 104 can be a CT scanner that is configured to capture or generate CT scanned images of any suitable anatomical structures of any suitable medical patients. As even another non-limiting example, the medical imaging scanner 104 can be a PET scanner that is configured to capture or generate PET scanned images of any suitable anatomical structures of any suitable medical patients. As yet another non-limiting example, the medical imaging scanner 104 can be an NM scanner that is configured to capture or generate NM scanned images of any suitable anatomical structures of any suitable medical patients. As still another non-limiting example, the medical imaging scanner 104 can be an ultrasound scanner that is configured to capture or generate ultrasound scanned images of any suitable anatomical structures of any suitable medical patients. As another non-limiting example, the medical imaging scanner 104 can be an MRI scanner that is configured to capture or generate MRI scanned images of any suitable anatomical structures of any suitable medical patients. Although not explicitly shown in the figures, the medical imaging scanner 104 can be electronically integrated with any suitable human-computer interface device, which can be remote from or local to the medical imaging scanner 104. Accordingly, a user or technician associated with the medical imaging scanner 104 can interact with or otherwise control the medical imaging scanner 104. Some non-limiting examples of the human-computer interface device can be a keyboard of the medical imaging scanner 104, a keypad of the medical imaging scanner 104, a touchscreen of the medical imaging scanner 104, or a voice-command system of the medical imaging scanner 104.

In various embodiments, the medical image 106 can be any suitable image exhibiting any suitable format, size, or dimensionality that is captured or generated by the medical imaging scanner 104. As a non-limiting example, the medical image 106 can be an x-by-y array of pixels, for any suitable positive integers *x* and *y.* As another non-limiting example, the medical image 106 can be an *x*-by-*y*-by-*z* array of voxels, for any suitable positive integers x, *y*, and *z*. In various aspects, the medical image 106 can visually depict or illustrate any suitable anatomical structures or surgical implants of any suitable medical patient. In various instances, an anatomical structure can be any suitable bodily organ of the medical patient, any suitable bodily tissue of the medical patient, any suitable body part of the medical patient, any suitable bodily fluid of the medical patient, any suitable bodily cavity of the medical patient, or any suitable portion thereof. In various cases, a surgical implant can be any suitable medical hardware (e.g., medical tubing, medical stitches, medical stents, pacemakers, medical rods, medical plates, medical screws) that can be surgically implanted or otherwise inserted into or near any suitable anatomical structure of the medical patient. However, in other cases, the medical image 106 can be a test image or phantom image that is visually empty or that otherwise does not depict any anatomical structure of any medical patient. In various instances, the medical image 106 can have undergone any suitable image reconstruction techniques, such as filtered back projection. In various cases, the medical image 106 can have undergone any other suitable pre-processing or post-processing techniques, such as reorientation, denoising, or resolution enhancement.

In various embodiments, the digital twin 108 can be any suitable collection or set of any suitable mathematical or physics-based models that can collectively simulate, forecast, or otherwise predict any suitable behavioral detail or aspect of the medical imaging scanner 104. As a non-limiting example, the digital twin 108 can comprise any suitable mass continuity equations, inequalities, or formulas that pertain in some way to the medical imaging scanner 104. As another non-limiting example, the digital twin 108 can comprise any suitable energy balance equations, inequalities, or formulas that pertain in some way to the medical imaging scanner 104. As even another non-limiting example, the digital twin 108 can comprise any suitable heat transfer equations, inequalities, or formulas that pertain in some way to the medical imaging scanner 104. As still another non-limiting example, the digital twin 108 can comprise any suitable fluid flow equations, inequalities, or formulas that pertain in some way to the medical imaging scanner 104. As yet another non-limiting example, the digital twin 108 can comprise any suitable kinetic or kinematic equations, inequalities, or formulas that pertain in some way to the medical imaging scanner 104. As another non-limiting example, the digital twin 108 can comprise any suitable Newtonian mechanics or quantum mechanics equations, inequalities, or formulas that pertain in some way to the medical imaging scanner 104. As still another non-limiting example, the digital twin 108 can comprise any suitable corrosion or degradation equations, inequalities, or formulas that pertain in some way to the medical imaging scanner 104.

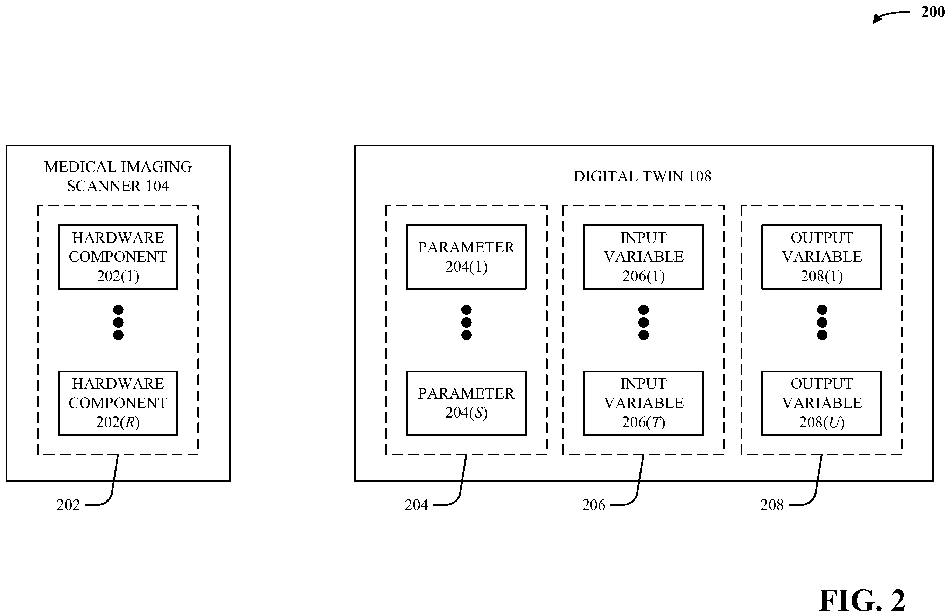

FIG. 2 illustrates an example, non-limiting block diagram showing the medical imaging scanner 104 and the digital twin 108 in accordance with one or more embodiments described herein.

In various embodiments, as shown, the medical imaging scanner 104 can be constructed from, be made up of, or otherwise comprise a plurality of hardware components 202. In various aspects, the plurality of hardware components 202 can comprise r components, for any suitable positive integer r > 1: a hardware component 202(1) to a hardware component *202(r).* In various instances, each of the plurality of hardware components 202 can be a respective, granular piece of active (e.g., actuatable, such as a motor) or passive (e.g., non-actuatable, such as an insulator) constituent hardware of the medical imaging scanner 104. As some non-limiting examples, any of the plurality of hardware components 202 can be: any suitable portion of a scanning detector of the medical imaging scanner 104 (e.g., can be a scintillator, photodetector, cell array, amplifier, or analog-to-digital converter); any suitable portion of an X-ray tube of the medical imaging scanner 104 (e.g., can be an anode, cathode, insulator, vacuum seal or enclosure, glass or metal envelope, electrode, electrode filament, focusing cup, anode rotor, radiation filter, or collimator/lens); any suitable portion of a gantry of the medical imaging scanner 104 (e.g., can be a gantry motor, gantry gear, gantry belt, or gantry bearing); any suitable portion of an actuatable patient table of the medical imaging scanner 104 (e.g., can be a table motor, table gear, table belt, or table bearing); or any suitable portion of a cooling system of the medical imaging scanner 104 (e.g., can be an oil immersion tank, heat pump, cooling fan, or heat exchanger). It should be appreciated that each of the plurality of hardware components 202 can be considered as having a respective unique identifier and as being positioned at a respective unique location within the medical imaging scanner 104.

In various embodiments, the digital twin 108 can be considered as a collection or set of mathematical or physics-based models that can simulate, forecast, or predict something about the medical imaging scanner 104 or about the plurality of hardware components 202, and those mathematical or physics-based models can be considered as being made up of a parametric state 204, a set of input variables 206, and a set of output variables 208. In particular, the parametric state 204 can be considered as defining the operators or coefficients of those mathematical or physics-based models, the set of input variables 206 can be considered as the operands or arguments of those mathematical or physics-based models, and the set of output variables 208 can be considered as the simulated, predicted, or forecasted results computed by those mathematical or physics-based models.

In various aspects, the parametric state 204 of the digital twin 108 can comprise s parameters, for any suitable positive integer s: a parameter 204(1) to a parameter 204(s). In various cases, each parameter of the parametric state 204 can be any suitable mathematical quantity that can represent a respective physical or theoretical characteristic, attribute, or property of the medical imaging scanner 104. For instance, the parameter 204(1) can be a scalar, a vector, a matrix, a tensor, or any suitable combination thereof that can represent a first physical or theoretical characteristic, attribute, or property of the medical imaging scanner 104. Likewise, the parameter 204(s) can be a scalar, a vector, a matrix, a tensor, or any suitable combination thereof that can represent an s-th physical or theoretical characteristic, attribute, or property of the medical imaging scanner 104. As some non-limiting examples, any parameter of the parametric state 204 can represent: a length of the medical imaging scanner 104 or of any portion or component thereof; a width of the medical imaging scanner 104 or of any portion or component thereof; a height of the medical imaging scanner 104 or of any portion or component thereof; a thickness of the medical imaging scanner 104 or of any portion or component thereof; a radius of curvature of the medical imaging scanner 104 or of any portion or component thereof; a mass or density of the medical imaging scanner 104 or of any portion or component thereof; a stiffness of the medical imaging scanner 104 or of any portion or component thereof; a damping coefficient of the medical imaging scanner 104 or of any portion or component thereof; an electrical resistance of the medical imaging scanner 104 or of any portion or component thereof; an electrical impedance of the medical imaging scanner 104 or of any portion or component thereof; a thermal resistance of the medical imaging scanner 104 or of any portion or component thereof; a thermal conductivity of the medical imaging scanner 104 or of any portion or component thereof; a heat capacity of the medical imaging scanner 104 or of any portion or component thereof; an optical opaqueness of the medical imaging scanner 104 or of any portion or component thereof; an optical aberration coefficient of the medical imaging scanner 104 or of any portion or component thereof; a decoherence time of the medical imaging scanner 104 or of any portion or component thereof; or a quantum Hamilton element of the medical imaging scanner 104 or of any portion or component thereof.

In some cases, any parameter of the parametric state 204 can represent a physical or theoretical characteristic, attribute, or property that is expected to be intransient, constant, or fixed. That is, the value of such physical or theoretical characteristic, attribute, or property can be expected to not drift, decay, or otherwise change over time or with use of the medical imaging scanner 104. However, in other cases, any parameter of the parametric state 204 can represent a physical or theoretical characteristic, attribute, or property that is expected to be transient, unfixed, or not constant. That is, the value of such physical or theoretical characteristic, attribute, or property can be expected to slowly or quickly drift, decay, or otherwise change over time or with use of the medical imaging scanner 104.

In various aspects, the set of input variables 206 of the digital twin 108 can comprise *t* variables, for any suitable positive integer *t:* an input variable 206(1) to an input variable 206(t). In various cases, each of the set of input variables 206 can be any suitable mathematical quantity that can represent any suitable dimension, feature, detail, or aspect of a usage scenario that the medical imaging scanner 104 can encounter or experience. For instance, the input variable 206(1) can be a scalar, a vector, a matrix, a tensor, or any suitable combination thereof that can represent a first dimension, feature, detail, or aspect of a usage scenario that is encounterable by the medical imaging scanner 104. Likewise, the input variable 206(t) can be a scalar, a vector, a matrix, a tensor, or any suitable combination thereof that can represent a t-th dimension, feature, detail, or aspect of a usage scenario that is encounterable by the medical imaging scanner 104. As some non-limiting examples, any of the set of input variables 206 can represent: any suitable attributes of a medical patient or anatomical structure that the medical imaging scanner 104 can scan (e.g., patient or structure size, weight, density, chemical composition, temperature, or absorption coefficient); a warm-up time afforded or allocated to the medical imaging scanner 104 for the performance of one or more scans; a run time afforded or allocated to the medical imaging scanner 104 for the performance of one or more scans; a cooldown time afforded or allocated to the medical imaging scanner 104 for the performance of one or more scans; a maximum or minimum voltage level to be used by the medical imaging scanner 104 for the performance of one or more scans; or a maximum or minimum radiation level to be used by the medical imaging scanner 104 for the performance of one or more scans.

In various aspects, the set of output variables 208 of the digital twin 108 can comprise *u* variables, for any suitable positive integer *u:* an output variable 208(1) to an output variable 208(u). In various cases, each of the set of output variables 208 can be any suitable mathematical quantity that can represent any suitable characteristic, attribute, property, feature, detail, or behavioral aspect of the medical imaging scanner 104 or of any of the plurality of hardware components 202 that is desired to be simulated, forecasted, or predicted. For instance, the output variable 208(1) can be a scalar, a vector, a matrix, a tensor, or any suitable combination thereof that can represent a first simulated, forecasted, or predicted characteristic, attribute, property, feature, detail, or behavioral aspect of the medical imaging scanner 104. Likewise, the output variable 208(u) can be a scalar, a vector, a matrix, a tensor, or any suitable combination thereof that can represent a u-th simulated, forecasted, or predicted characteristic, attribute, property, feature, detail, or behavioral aspect of the medical imaging scanner 104. As a non-limiting example, any of the set of output variables 208 can represent an image (e.g., a pixel array or a voxel array) that is outputtable by the medical imaging scanner 104. As another non-limiting example, any of the set of output variables 208 can represent a total or marginal amount of degradation or wear that the medical imaging scanner 104, or any of the plurality of hardware components 202, can experience or accumulate in response to any given usage scenario. As yet another non-limiting example, any of the set of output variables 208 can represent a total or marginal amount of electricity that is consumable by the medical imaging scanner 104 in response to any given usage scenario. As even another non-limiting example, any of the set of output variables 208 can represent an amount of useful life (e.g., expressed in hours or runs) of the medical imaging scanner 104 or of any of the plurality of hardware components 202 that will remain or be left in response to any given usage scenario.

Accordingly, the digital twin 108 can simulate, forecast, or predict how the medical imaging scanner 104 will respond to any particular usage scenario. Specifically, the set of input variables 206 can be assigned any suitable numerical values that correspond to or otherwise define that particular usage scenario, the parametric state 204 can be applied (e.g., according to whatever mathematical functions, operations, or formulas make up the digital twin 108) to the set of input variables 206, and the set of output variables 208 can be equal to the computational results (e.g., products, differences, sums, quotients) of such application.

Referring back to FIG. 1, it can be the case that the medical imaging scanner 104 lacks internal operability sensors, such that the operational or functional states of the plurality of hardware components 202 cannot be monitored in real-time. Despite such lack of internal operability sensors, it can nevertheless be desired to predict or forecast failures or malfunctions of the medical imaging scanner 104. As described herein, the fault system 102 can facilitate such prediction or forecasting.

In various embodiments, the fault system 102 can comprise a processor 110 (e.g., computer processing unit, microprocessor) and a non-transitory computer-readable memory 112 that is operably or operatively or communicatively connected or coupled to the processor 110. The non-transitory computer-readable memory 112 can store computer-executable instructions which, upon execution by the processor 110, can cause the processor 110 or other components of the fault system 102 (e.g., access component 114, failure component 116, cause component 118, life component 120, action component 122) to perform one or more acts. In various embodiments, the non-transitory computer-readable memory 112 can store computer-executable components (e.g., access component 114, failure component 116, cause component 118, life component 120, action component 122), and the processor 110 can execute the computer-executable components.

In various embodiments, the fault system 102 can comprise an access component 114. In various aspects, the access component 114 can electronically access or otherwise electronically communicate in any suitable fashion with the medical imaging scanner 104, with the medical image 106, or with the digital twin 108. For instance, the access component 114 can electronically transmit any suitable electronic data to, or receive any suitable electronic data from, the medical imaging scanner 104 or the digital twin 108. As another instance, the access component 114 can electronically receive or electronically retrieve the medical image 106 from any suitable electronic source (e.g., from the medical imaging scanner 104 itself). Accordingly, the access component 114 can be considered as a proxy or conduit by which other components of the fault system 102 can electronically interact with the medical imaging scanner 104, with the medical image 106, or with the digital twin 108.

In various embodiments, the fault system 102 can comprise a failure component 116. In various aspects, the failure component 116 can, as described herein, electronically generate a failure classification label for the medical image 106.

In various embodiments, the fault system 102 can comprise a cause component 118. In various aspects, the cause component 118 can, as described herein, electronically generate a root cause classification label for the medical image 106, based on the failure classification label.

In various embodiments, the fault system 102 can comprise a life component 120. In various aspects, the life component 120 can, as described herein, electronically estimate a remaining useful life for some hardware component of the medical imaging scanner 104, based on the failure classification label and the root cause classification label.

In various embodiments, the fault system 102 can comprise an action component 122. In various aspects, the action component 122 can, as described herein, electronically initiate any suitable actions, based on the failure classification label, based on the root cause classification label, or based on the estimated remaining useful life.

Note that, in various instances, the access component 114, the failure component 116, the cause component 118, the life component 120, and the action component 122 can collectively be considered as being one or more software components 113 of the fault system 102. In various aspects, it should be appreciated that the one or more software components 113 are described primarily herein as comprising five components (e.g., the access component 114, the failure component 116, the cause component 118, the life component 120, and the action component 122) for ease of explanation and illustration. However, the one or more software components 113 are not limited to being implemented as exactly such five components in every embodiment. Indeed, in some embodiments, the functionalities described herein of such five components can be combined in any suitable fashions, so as to be implemented in or by fewer than five components (e.g., in some cases, a single component can perform all of the functionalities that are described herein with respect to the access component 114, the failure component 116, the cause component 118, the life component 120, and the action component 122). In other embodiments, the functionalities described herein of such five components can instead be distributed, separated, split, or fragmented in any suitable fashions, so as to be implemented in or by more than five components (e.g., two or more components can facilitate the functionalities that are performable by the access component 114; two or more components can facilitate the functionalities that are performable by the failure component 116; two or more components can facilitate the functionalities that are performable by the cause component 118; two or more components can facilitate the functionalities that are performable by the life component 120; two or more components can facilitate the functionalities that are performable by the action component 122).

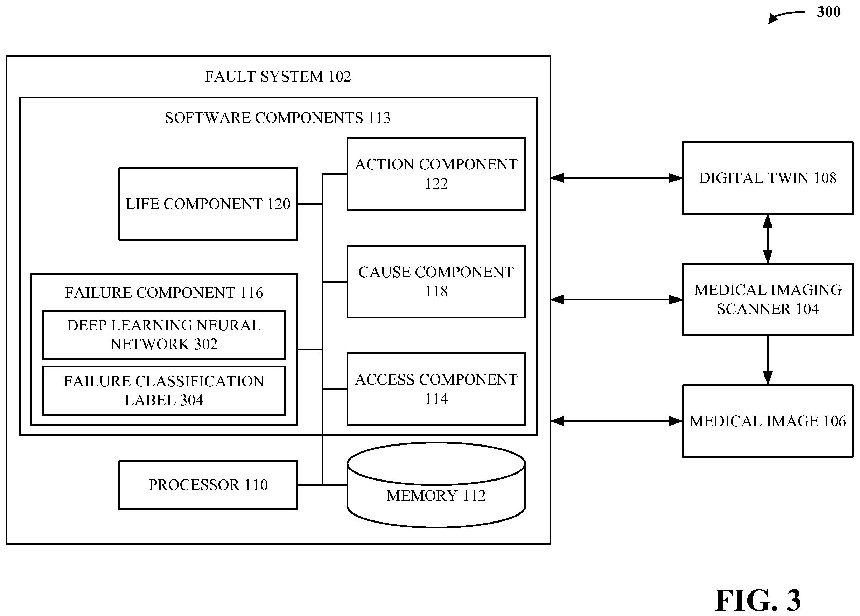

FIG. 3 illustrates a block diagram of an example, non-limiting system including a first deep learning neural network and a failure classification label that can facilitate scanner fault prediction via image-based deep learning in accordance with one or more embodiments described herein. As shown, the system 300 can, in some cases, comprise the same components as the system 100, and can further comprise a deep learning neural network 302 and a failure classification label 304.

In various embodiments, the failure component 116 can electronically store, electronically maintain, electronically control, or otherwise electronically access the deep learning neural network 302. In various aspects, the deep learning neural network 302 can exhibit any suitable deep learning internal architecture. Indeed, in various cases, the deep learning neural network 302 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

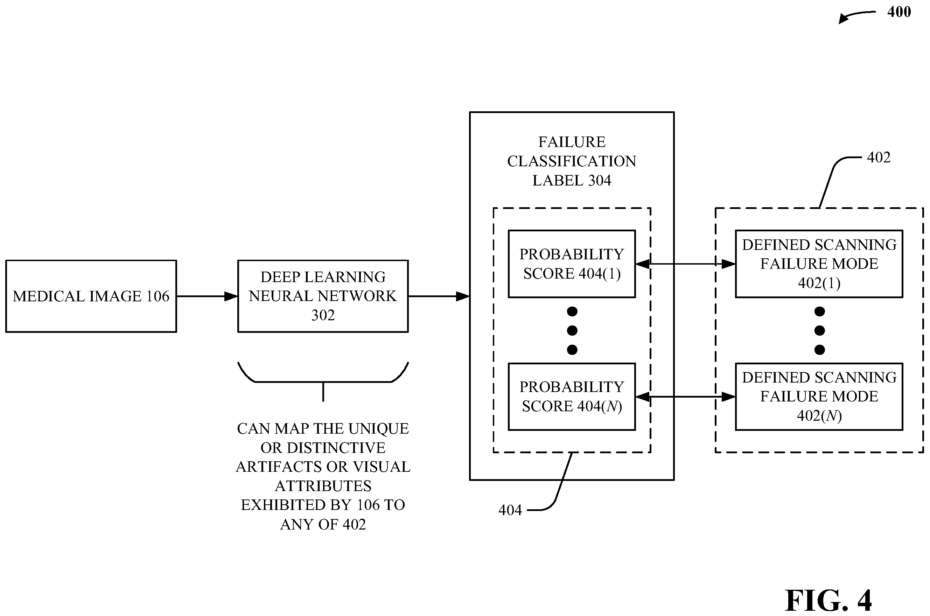

Regardless of its specific internal architecture (e.g., of its specific numbers, types, or organizations of layers), the deep learning neural network 302 can be configured as an image classifier. In various aspects, the failure component 116 can electronically leverage the deep learning neural network 302, so as to generate the failure classification label 304 based on the medical image 106. Non-limiting aspects are described with respect to FIG. 4.

FIG. 4 illustrates an example, non-limiting block diagram 400 showing how the deep learning neural network 302 can generate the failure classification label 304 in accordance with one or more embodiments described herein.

In various embodiments, the failure component 116 can electronically execute the deep learning neural network 302 on the medical image 106. In various aspects, such execution can cause the deep learning neural network 302 to produce the failure classification label 304. More specifically, the failure component 116 can feed the medical image 106 to the input layer of the deep learning neural network 302. In various cases, the medical image 106 can complete a forward pass through the one or more hidden layers of the deep learning neural network 302. In various aspects, the output layer of the deep learning neural network 302 can compute or calculate the failure classification label 304 based on activation maps or feature maps produced by the one or more hidden layers of the deep learning neural network 302.

In various aspects, the failure classification label 304 can be any suitable electronic data (e.g., can be one or more scalars, one or more vectors, one or more matrices, one or more tensors, one or more character strings, or any suitable combination thereof) that can represent, convey, or otherwise indicate a specific failure or fault that the medical imaging scanner 104 is (in the opinion of the deep learning neural network 302) experiencing or otherwise afflicted with. In particular, there can be a plurality of defined scanning failure modes 402. In various instances, the plurality of defined scanning failure modes 402 can comprise n modes, for any suitable positive integer n > 1: a defined scanning failure mode 402(1) to a defined scanning failure mode 402(*n*)*.* In various cases, each of the plurality of defined scanning failure modes 402 can be or otherwise represent a distinct or unique way (referred to as "mode") in which the imaging capabilities of the medical imaging scanner 104 can possibly fail. In some instances, each of the plurality of defined scanning failure modes 402 can be or otherwise represent a failure or malfunction of a respective coarse subsystem of the medical imaging scanner 104 that is made up of a subset of the plurality of hardware components 202. As a non-limiting example, any of the plurality of defined scanning failure modes 402 can be any suitable failure mode that pertains to radiation emission of the medical imaging scanner 104. For instance, an X-ray tube (which is responsible for emitting radiation) can be considered as an aggregate or coarse subsystem made up of multiple granular hardware components, and can fail in various ways, such as by experiencing tube arcing (e.g., a tube arcing failure mode) or by experiencing overheating (e.g., a tube overheating failure mode). As another non-limiting example, any of the plurality of defined scanning failure modes 402 can be any suitable failure mode that pertains to radiation detection of the medical imaging scanner 104. For instance, a detector (which is responsible for measuring radiation that has passed through a medical patient) can be considered as an aggregate or coarse subsystem made up of multiple granular hardware components, and can fail in various ways, such as by experiencing noise (e.g., a detector noise failure mode) or by experiencing drift (e.g., a detector drift failure mode). As yet another non-limiting example, any of the plurality of defined scanning failure modes 402 can be any suitable failure mode that pertains to mechanical operation or movement of the medical imaging scanner 104. For instance, a gantry (which can be responsible for physical reorienting X-ray tubes or detectors with respect to a medical patient) can be considered as an aggregate or coarse subsystem made up of multiple granular hardware components, and can fail in various ways, such as by experiencing misalignment (e.g., a gantry misalignment failure mode) or by experiencing unstable or rough movement (e.g., a gantry instability failure mode).

In various aspects, the failure classification label 304 can comprise a plurality of probability scores 404. In various instances, the plurality of probability scores 404 can respectively correspond (e.g., in one-to-one fashion) to the plurality of defined scanning failure modes 402. Thus, since the plurality of defined scanning failure modes 402 can comprise n modes, the plurality of probability scores 404 can likewise comprise n scores; a probability score 404(1) to a probability score 404(*n*)*.* In various cases, each of the plurality of probability scores 404 can be a real-valued scalar that indicates a likelihood (as inferred by the deep learning neural network 302) that the medical imaging scanner 104 is experiencing or otherwise afflicted with a respective one of the plurality of defined scanning failure modes 402. As a non-limiting example, the probability score 404(1) can correspond to the defined scanning failure mode 402(1). Thus, the probability score 404(1) can be a first scalar estimated by the deep learning neural network 302 and whose value (e.g., ranging from 0 to 1, or from 0% to 100%) indicates a likelihood that the medical imaging scanner 104 is currently or presently afflicted with the defined scanning failure mode 402(1). As another non-limiting example, the probability score 404(*n*) can correspond to the defined scanning failure mode 402(*n*)*.* So, the probability score 404(*n*) can be an n-th scalar estimated by the deep learning neural network 302 and whose value indicates a likelihood that the medical imaging scanner 104 is currently or presently afflicted with the defined scanning failure mode 402(*n*)*.*

Although not explicitly shown in FIG. 4, it should be appreciated that the failure classification label 304 can include an (n + 1)-th probability score which can indicate a likelihood (as inferred by the deep learning neural network 302) that the medical imaging scanner 104 is not currently or presently afflicted with any of the plurality of defined scanning failure modes 402.

Note that, in some cases, the plurality of probability scores 404 can be not independent of each other. As a non-limiting example, the plurality of probability scores 404 can be restricted such that their total sum (including the (n + 1)-th probability score if applicable) can be unity (e.g., can be 1 or 100%). In such case, the deep learning neural network 302 can be considered as determining that the medical imaging scanner 104 is currently or presently afflicted with at most one of the plurality of defined scanning failure modes 402 (e.g., whichever one of the plurality of defined scanning failure modes 402 has the highest probability score can be considered as being indicated by the failure classification label 304). However, in other cases, the plurality of probability scores 404 can be independent of each other. As a non-limiting example, each of the plurality of probability scores 404 can range from 0 (e.g., 0%) to 1 (e.g., 100%), regardless of the values of any others of the plurality of probability scores 404 (e.g., with no unity restriction on the total sum of the plurality of probability scores 404). In such case, the deep learning neural network 302 can be considered as being able to determine that the medical imaging scanner 104 is currently or presently afflicted with any number of the plurality of defined scanning failure modes 402. In other words, the deep learning neural network 302 can infer that the medical imaging scanner 104 is afflicted with multiple defined scanning failure modes at once (e.g., whichever one or more of the plurality of defined scanning failure modes 402 have probability scores that exceed any suitable threshold value can be considered as being indicated by the failure classification label 304). Note that, even in situations where the plurality of probability scores 404 are constrained to sum to unity, the failure classification label 304 can nevertheless sometimes be interpreted to indicate more than one of the plurality of defined scanning failure modes 402 (e.g., the failure classification label 304 can be considered as indicating whichever h of the plurality of defined scanning failure modes 402 have the highest probability scores, for any suitable positive integer *h* < *n*)*.*

In any case, the medical image 106 can be considered as having, displaying, illustrating, or otherwise exhibiting any suitable unique or distinct visual attributes (e.g., unique or distinct visual artifacts, unique or distinct blurriness or distortion pattern), which may or may not be noticeable or apparent to the naked eye, and the deep learning neural network 302 can be considered as mapping or correlating those unique or distinct visual attributes to respective ones of the plurality of defined scanning failure modes 402. In other words, each of the plurality of defined scanning failure modes 402 could, if it were to actually afflict or otherwise occur with respect to the medical imaging scanner 104, manifest itself via a unique visual fingerprint or signature within the medical image 106. As a non-limiting example, a tube arcing failure mode can cause the medical image 106 to have some first visual fingerprint or signature (e.g., some first type of artifacts or distortions). In contrast, a tube overheating failure mode can cause the medical image 106 to have some second visual fingerprint or signature that is different from the first visual fingerprint or signature. Furthermore, a gantry misalignment failure mode can cause the medical image 106 to have some third visual fingerprint or signature that is different from both the first and second visual fingerprints or signatures. In any case, the deep learning neural network 302 can, due to its training described later herein, be able to detect or recognize whatever unique visual fingerprints or signatures are within the medical image 106, and the deep learning neural network 302 can accordingly infer or predict which one or more of the plurality of defined scanning failure modes 402 are currently or presently afflicting the medical imaging scanner 104.

Note that, in some aspects, the plurality of probability scores 404 can be considered as indicating respective severity levels of the plurality of defined scanning failure modes 402. Indeed, as the severity of a particular defined scanning failure mode that the medical imaging scanner 104 is afflicted with increases, the visual fingerprint or signature of that particular defined scanning failure mode can become progressively more apparent or conspicuous within the medical image 106, and the deep learning neural network 302 can accordingly become progressively more sure that the medical imaging scanner 104 is afflicted with the particular defined scanning failure mode. In this way, a high probability score can be considered as indicating a high severity level, whereas a low probability score can be considered as indicating a low severity level.

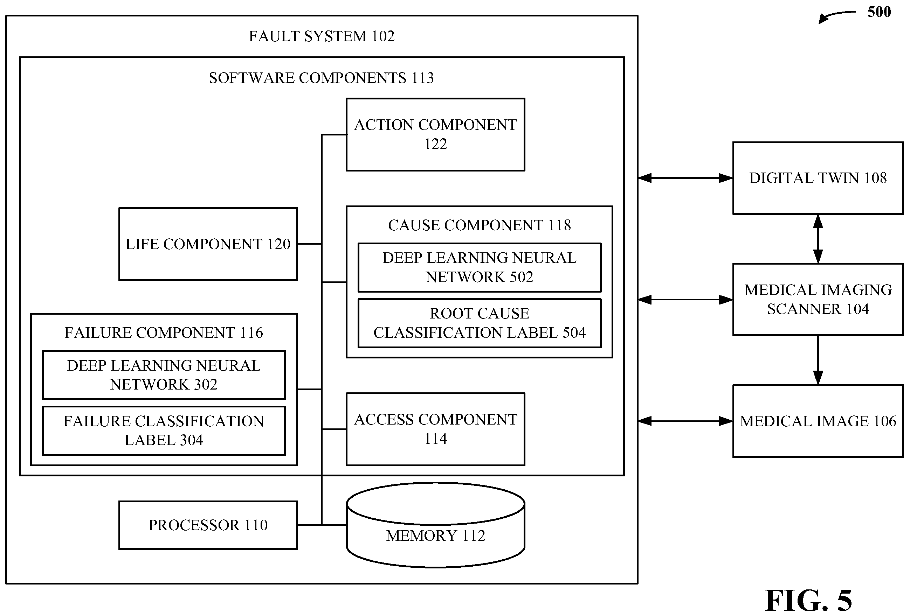

FIG. 5 illustrates a block diagram of an example, non-limiting system 500 including a second deep learning neural network and a root cause classification label that can facilitate scanner fault prediction via image-based deep learning in accordance with one or more embodiments described herein. As shown, the system 500 can, in some cases, comprise the same components as the system 300, and can further comprise a deep learning neural network 502 and a root cause classification label 504.

In various embodiments, the cause component 118 can electronically store, electronically maintain, electronically control, or otherwise electronically access the deep learning neural network 502. In various aspects, the deep learning neural network 502 can exhibit any suitable deep learning internal architecture. Indeed, in various cases, the deep learning neural network 502 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

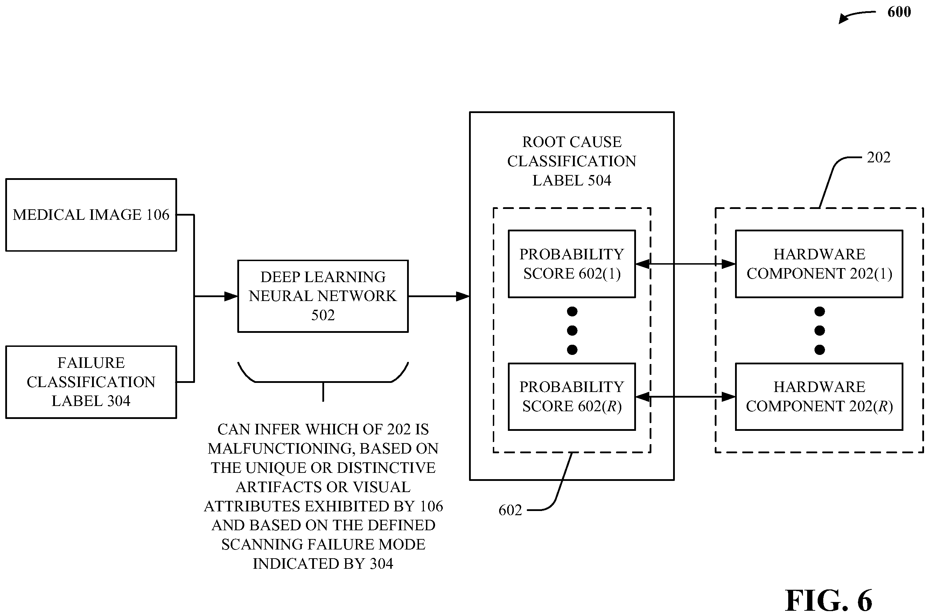

Regardless of its specific internal architecture, the deep learning neural network 502 can be configured as an image classifier that is conditioned on the output of the deep learning neural network 302. In various aspects, the cause component 118 can, in response to the failure classification label 304 indicating that the medical imaging scanner 104 is afflicted by at least one of the plurality of defined scanning failure modes, electronically leverage the deep learning neural network 502, so as to generate the root cause classification label 504 based on the medical image 106 and based on the failure classification label 304. Non-limiting aspects are described with respect to FIG. 6.

FIG. 6 illustrates an example, non-limiting block diagram 600 showing how the deep learning neural network 502 can generate the root cause classification label 504 in accordance with one or more embodiments described herein.

In various embodiments, the cause component 118 can electronically execute the deep learning neural network 502 on the medical image 106 and on the failure classification label 304. In various aspects, such execution can cause the deep learning neural network 502 to produce the root cause classification label 504. More specifically, the cause component 118 can concatenate the medical image 106 and the failure classification label 304 together. In various instances, the cause component 118 can feed that concatenation to the input layer of the deep learning neural network 502. In various cases, that concatenation can complete a forward pass through the one or more hidden layers of the deep learning neural network 502. In various aspects, the output layer of the deep learning neural network 502 can compute or calculate the root cause classification label 504 based on activation maps or feature maps produced by the one or more hidden layers of the deep learning neural network 502.

In various aspects, the root cause classification label 504 can be any suitable electronic data (e.g., can be one or more scalars, one or more vectors, one or more matrices, one or more tensors, one or more character strings, or any suitable combination thereof) that can represent, convey, or otherwise indicate a specific hardware component of the medical imaging scanner 104 that is (in the opinion of the deep learning neural network 502) malfunctioning and thereby causing whatever defined scanning failure mode is indicated by the failure classification label 304. In particular, the root cause classification label 504 can comprise a plurality of probability scores 602. In various instances, the plurality of probability scores 602 can respectively correspond (e.g., in one-to-one fashion) to the plurality of hardware components 202. Thus, since the plurality of hardware components 202 can comprise r components, the plurality of probability scores 602 can likewise comprise r scores; a probability score 602(1) to a probability score 602(r). In various cases, each of the plurality of probability scores 602 can be a real-valued scalar that indicates a likelihood (as inferred by the deep learning neural network 502) that a respective one of the plurality of hardware components 202 that make up the medical imaging scanner 104 is malfunctioning, damaged, degraded, or otherwise not operating as expected. As a non-limiting example, the probability score 602(1) can correspond to the hardware component 202(1). Thus, the probability score 602(1) can be a first scalar estimated by the deep learning neural network 502 and whose value (e.g., ranging from 0 to 1, or from 0% to 100%) indicates a likelihood that the hardware component 202(1) is broken or malfunctioning. Stated differently, the probability score 602(1) can be a first scalar estimated by the deep learning neural network 502 and whose value indicates a likelihood that the hardware component 202(1) is responsible for or otherwise causing whichever defined scanning failure mode that is indicated by the failure classification label 304. As another non-limiting example, the probability score 602(r) can correspond to the hardware component *202(r).* So, the probability score 602(r) can be an r-th scalar estimated by the deep learning neural network 502 and whose value indicates a likelihood that the hardware component *202(r)* is broken or malfunctioning. Stated differently, the probability score 602(r) can be an r-th scalar estimated by the deep learning neural network 502 and whose value indicates a likelihood that the hardware component *202(r)* is responsible for or otherwise causing whichever defined scanning failure mode that is indicated by the failure classification label 304.

Note that, in some cases, the plurality of probability scores 602 can be not independent of each other. As a non-limiting example, the plurality of probability scores 602 can be restricted such that their total sum can be unity (e.g., can be 1 or 100%). In such case, the deep learning neural network 502 can be considered as determining that exactly one of the plurality of hardware components 202 is malfunctioning and thus responsible for whichever defined scanning failure mode is indicated by the failure classification label 304 (e.g., whichever one of the plurality of hardware components 202 has the highest probability score can be considered as being indicated by the root cause classification label 504). However, in other cases, the plurality of probability scores 602 can be independent of each other. As a non-limiting example, each of the plurality of probability scores 602 can range from 0 (e.g., 0%) to 1 (e.g., 100%), regardless of the values of any others of the plurality of probability scores 602 (e.g., with no unity restriction on the total sum of the plurality of probability scores 602). In such case, the deep learning neural network 502 can be considered as being able to determine that more than one of the plurality of hardware components 202 are malfunctioning and thus jointly responsible for whichever defined scanning failure mode is indicated by the failure classification label 304 (e.g., whichever one or more of the plurality of hardware components 202 have probability scores that exceed any suitable threshold value can be considered as being indicated by the root cause classification label 504). As above, note that, even in situations where the plurality of probability scores 602 are constrained to sum to unity, the root cause classification label 504 can nevertheless sometimes be interpreted to indicate more than one of the plurality of hardware components 202 (e.g., the root cause classification label 504 can be considered as indicating whichever g of the plurality of hardware components 202 have the highest probability scores, for any suitable positive integer *g* < *r*).

In any case, the medical image 106 can, as mentioned above, be considered as having, displaying, illustrating, or otherwise exhibiting any suitable unique or distinct visual attributes, which may or may not be noticeable or apparent to the naked eye. As also mentioned above, the deep learning neural network 302 can be considered as mapping or correlating those unique or distinct visual attributes to respective ones of the plurality of defined scanning failure modes 402. Now, in various aspects, the deep learning neural network 502 can instead be considered as mapping or correlating those unique or distinct visual attributes, as conditioned by or supplemented with the failure classification label 304, to respective ones of the plurality of hardware components 202. More specifically, just as each of the plurality of defined scanning failure modes 402 could potentially manifest itself via a unique visual fingerprint or signature within the medical image 106, the malfunctioning of each of the plurality of hardware components 202 could likewise potentially manifest itself via a unique visual fingerprint or signature within the medical image 106.

As a non-limiting example, suppose that the failure classification label 304 indicates that the medical imaging scanner 104 is afflicted with a tube arcing failure mode. That is, the deep learning neural network 302 can have detected or recognized within the medical image 106 visual evidence (e.g., inconspicuous or conspicuous artifacts or distortions) that is unique to or characteristic of tube arcing. Tube arcing can have various different root causes, each of which can be considered as leaving its own unique visual fingerprint or signature in the medical image 106. For instance, an X-ray tube can experience tube arcing when its insulator degrades, breaks-down, or otherwise malfunctions. However, the X-ray tube can alternatively experience tube arcing when its vacuum enclosure degrades, breaks-down, or otherwise malfunctions. Tube arcing that is caused by a faulty insulator can cause the medical image 106 to have a visual fingerprint/signature A. In contrast, tube arcing that is caused by a faulty vacuum enclosure can cause the medical image 106 to have a visual fingerprint/signature B that is different from the visual fingerprint/signature A.

As another non-limiting example, suppose that the failure classification label 304 indicates that the medical imaging scanner 104 is afflicted with a tube overheating failure mode. That is, the deep learning neural network 302 can have detected or recognized within the medical image 106 visual evidence that is unique to or characteristic of tube overheating. As above, tube overheating can have various different root causes, each of which can be considered as leaving its own unique visual fingerprint or signature in the medical image 106. For instance, an X-ray tube can experience overheating of its anode. However, the X-ray tube can alternatively experience overheating of its cathode. Anode overheating can cause the medical image 106 to have a visual fingerprint/signature C. In contrast, cathode overheating can cause the medical image 106 to have a visual fingerprint/signature D that is different from the visual fingerprint/signature C.

As yet another non-limiting example, suppose that the failure classification label 304 indicates that the medical imaging scanner 104 is afflicted with a beam strength instability failure mode. That is, the deep learning neural network 302 can have detected or recognized within the medical image 106 visual evidence that is unique to or characteristic of instability, unevenness, or other irregularities in the strength or energy level of the radiation beams emitted by an X-ray tube of the medical imaging scanner 104. Just as above, beam strength instability can have various different root causes, each of which can be considered as leaving its own unique visual fingerprint or signature in the medical image 106. For instance, an X-ray tube can experience beam strength instability due to pitting or cracking of its anode. However, the X-ray tube can alternatively experience beam strength instability due to burn-out or breakage of its filament. Beam strength instability caused by anode pitting/cracking can cause the medical image 106 to have a visual fingerprint/signature E. In contrast, beam strength instability caused by filament burn-out/breakage can cause the medical image 106 to have a visual fingerprint/signature F that is different from the visual fingerprint/signature E.

As even another non-limiting example, suppose that the failure classification label 304 indicates that the medical imaging scanner 104 is afflicted with a gantry instability failure mode. That is, the deep learning neural network 302 can have detected or recognized within the medical image 106 visual evidence that is unique to or characteristic of mechanical unsteadiness of the gantry of the medical imaging scanner 104. Just as above, gantry instability can have various different root causes, each of which can be considered as leaving its own unique visual fingerprint or signature in the medical image 106. For instance, the gantry can experience mechanical instability or unsteadiness due to malfunctioning of its motor. However, the gantry can alternatively experience mechanical instability or unsteadiness due to malfunctioning of its bearings. Gantry instability caused by motor malfunctioning can cause the medical image 106 to have a visual fingerprint/signature G. In contrast, gantry instability caused by bearing malfunctioning can cause the medical image 106 to have a visual fingerprint/signature H that is different from the visual fingerprint/signature G.

As still another non-limiting example, suppose that the failure classification label 304 indicates that the medical imaging scanner 104 is afflicted with an optical obfuscation failure mode. That is, the deep learning neural network 302 can have detected or recognized within the medical image 106 visual evidence that is unique to or characteristic of optical obfuscation of the medical imaging scanner 104. Just as above, optical obfuscation can have various different root causes, each of which can be considered as leaving its own unique visual fingerprint or signature in the medical image 106. For instance, optical obfuscation can be caused by condensation, dust, or other debris accumulating on a detector of the medical imaging scanner 104. However, optical obfuscation can alternatively be caused by condensation, dust, or other debris accumulating on a focusing lens or aperture of the medical imaging scanner 104. Detector-side optical obfuscation can cause the medical image 106 to have a visual fingerprint/signature I. In contrast, lens-side optical obfuscation can cause the medical image 106 to have a visual fingerprint/signature J that is different from the visual fingerprint/signature I.

In any case, the deep learning neural network 502 can, due to its training described later herein, be able to detect or recognize whatever unique visual fingerprints or signatures are within the medical image 106 as supplemented by the failure classification label 304, and the deep learning neural network 502 can accordingly infer or predict which one or more of the plurality of hardware components 202 are currently or presently malfunctioning or otherwise not operating correctly in the medical imaging scanner 104.

Note that an accuracy level of the deep learning neural network 502 can be considered as being increased or heightened, due to the fact that the deep learning neural network 502 can be conditioned on (e.g., can receive as input) the failure classification label 304. Indeed, as mentioned above, whichever defined scanning failure mode is indicated by the failure classification label 304 can be considered as being or identifying a coarse subsystem of the medical imaging scanner 104 that is made up of some respective subset of the plurality of hardware components 202. For example, a tube arcing failure mode can correspond to an X-ray tube of the medical imaging scanner 104, and an X-ray tube can be considered as a coarse subsystem made up of various granular hardware components (e.g., anodes, cathodes, filaments, vacuum seals). As another non-limiting example, a gantry instability failure mode can correspond to a gantry of the medical imaging scanner 104, and a gantry can be considered as a coarse subsystem made up of various granular hardware components (e.g., motors, bearings). So, for whatever coarse subsystem corresponds to the defined scanning failure mode indicated by the failure classification label 304, the deep learning neural network 502 can be considered as inferring which granular hardware component within that coarse subsystem is malfunctioning. In other words, because the deep learning neural network 502 can receive as input the output produced by the deep learning neural network 302, the inferencing work performed by the deep learning neural network 302 can be considered as restricting or shrinking the inferencing space or search space of the deep learning neural network 502. In still other words, the inferencing work performed by the deep learning neural network 302 can be considered as helping, assisting, or otherwise supplementing the deep learning neural network 502 in the performance of its own inferencing work.

Note that, in some aspects, the plurality of probability scores 602 can be considered as indicating respective malfunction severity levels of the plurality of hardware components 202. Indeed, as the malfunctioning severity of a particular hardware component increases, the visual fingerprint or signature that is unique to the malfunctioning of that particular hardware component can become progressively more apparent or conspicuous within the medical image 106, and the deep learning neural network 502 can accordingly become progressively more sure that the particular hardware component is malfunctioning. In this way, a high probability score can be considered as indicating a high malfunctioning severity level, whereas a low probability score can be considered as indicating a low malfunctioning severity level.

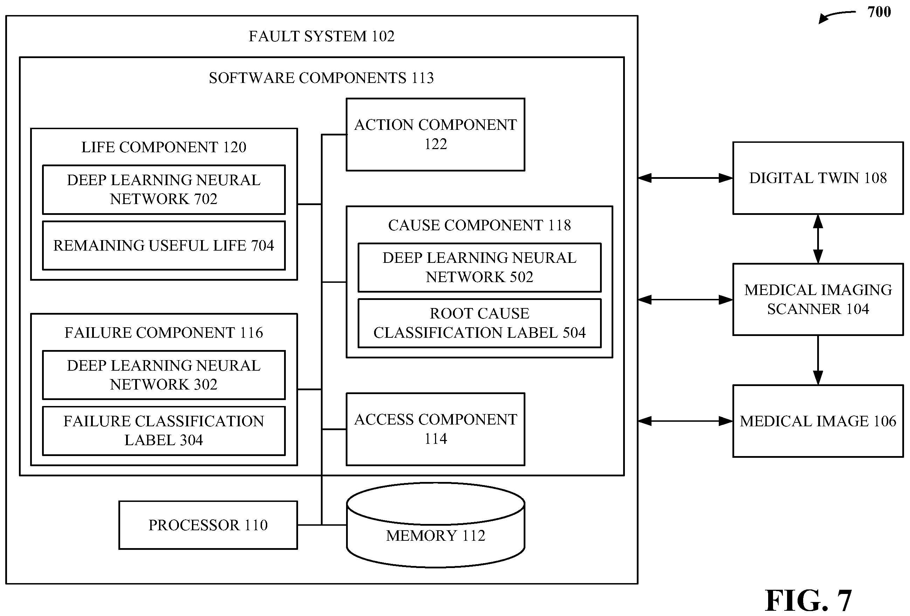

FIG. 7 illustrates a block diagram of an example, non-limiting system 700 including a third deep learning neural network and a remaining useful life that can facilitate scanner fault prediction via image-based deep learning in accordance with one or more embodiments described herein. As shown, the system 700 can, in some cases, comprise the same components as the system 500, and can further comprise a deep learning neural network 702 and a remaining useful life 704 (hereafter "RUL 704").

In various embodiments, the life component 120 can electronically store, electronically maintain, electronically control, or otherwise electronically access the deep learning neural network 702. In various aspects, the deep learning neural network 702 can exhibit any suitable deep learning internal architecture. Indeed, in various cases, the deep learning neural network 702 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

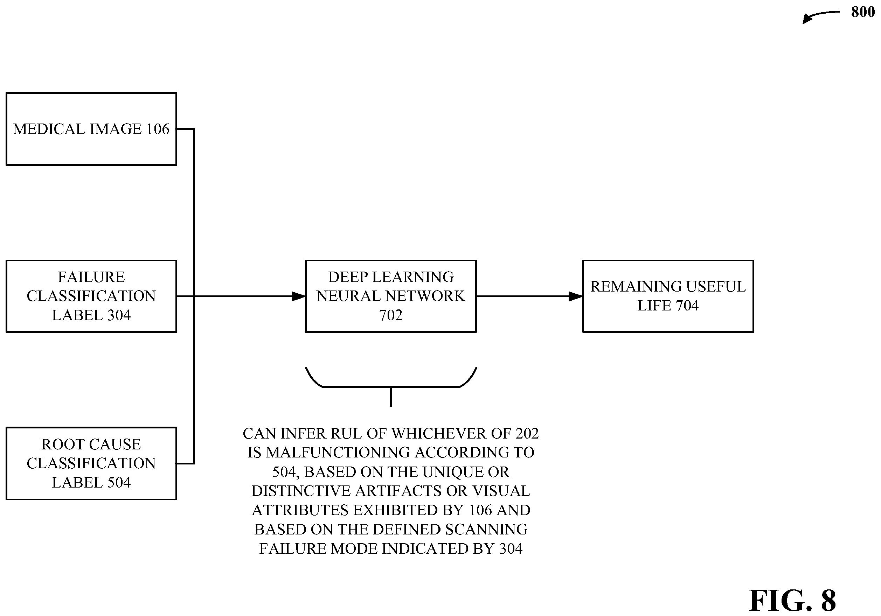

Regardless of its specific internal architecture, the deep learning neural network 702 can be configured as an image regressor that is conditioned on the outputs of the deep learning neural network 302 and the deep learning neural network 502. In various aspects, the life component 120 can electronically leverage the deep learning neural network 702, so as to generate the RUL 704 based on the medical image 106, based on the failure classification label 304, and based on the root cause classification label 504. Non-limiting aspects are described with respect to FIG. 8.

FIG. 8 illustrates an example, non-limiting block diagram 800 showing how the deep learning neural network 702 can generate the RUL 704 in accordance with one or more embodiments described herein.

In various embodiments, the life component 120 can electronically execute the deep learning neural network 702 on the medical image 106, on the failure classification label 304, and on the root cause classification label 504. In various aspects, such execution can cause the deep learning neural network 702 to produce the RUL 704. More specifically, the life component 120 can concatenate the medical image 106, the failure classification label 304, and the root cause classification label 504 together. In various instances, the life component 120 can feed that concatenation to the input layer of the deep learning neural network 702. In various cases, that concatenation can complete a forward pass through the one or more hidden layers of the deep learning neural network 702. In various aspects, the output layer of the deep learning neural network 702 can compute or calculate the RUL 704 based on activation maps or feature maps produced by the one or more hidden layers of the deep learning neural network 702.

In various aspects, the RUL 704 can be a real-valued scalar whose magnitude indicates an amount of useful life that remains or is left (as inferred by the deep learning neural network 702) for whichever hardware component is indicated by the root cause classification label 504. In some cases, the RUL 704 can be expressed in terms of remaining hours of operation for which such hardware component can be used. In other cases, the RUL 704 can instead be expressed in terms of remaining number of scans for which such hardware component can be used. However, these are mere non-limiting examples. In various aspects, the RUL 704 can exhibit any other suitable format, size, dimensionality, or units of measurement. As a non-limiting example, the RUL 704 can, in some cases, be a categorical variable that can indicate one of two or more possible RUL categories or states. For instance, such possible RUL categories or states can include: a "0 to 7 days remaining" category or state; a "7 to 21 days remaining" category or state; a "21 to 60 days remaining" category or state; or a "more than 60 days remaining" category or state.

In any case, the medical image 106 can, as mentioned above, be considered as having, displaying, illustrating, or otherwise exhibiting any suitable unique or distinct visual attributes, which may or may not be noticeable or apparent to the naked eye. As also mentioned above, the deep learning neural network 302 can be considered as mapping or correlating those unique or distinct visual attributes to respective ones of the plurality of defined scanning failure modes 402. As yet also mentioned above, the deep learning neural network 502 can be considered as mapping or correlating those unique or distinct visual attributes, as conditioned by or supplemented with the failure classification label 304, to respective ones of the plurality of hardware components 202. Now, in various aspects, the deep learning neural network 702 can instead be considered as mapping or correlating those unique or distinct visual attributes, as conditioned by or supplemented with both the failure classification label 304 and the root cause classification label 504, to remaining useful life estimations. More specifically, just as each of the plurality of defined scanning failure modes 402 could potentially manifest itself via a unique visual fingerprint or signature within the medical image 106, and just as the malfunctioning of each of the plurality of hardware components 202 could potentially manifest itself via a unique visual fingerprint or signature within the medical image 106, the amount of useful life or time that a particular malfunctioning hardware component has left can likewise manifest itself via a unique visual fingerprint or signature within the medical image 106.

As a non-limiting example, suppose that the failure classification label 304 indicates that the medical imaging scanner 104 is afflicted with a tube arcing failure mode, and suppose that the root cause classification label 504 indicates that an insulator of the medical imaging scanner 104 is malfunctioning and therefore causing tube arcing. That is, the deep learning neural network 302 can have detected or recognized within the medical image 106 visual evidence that is unique to or characteristic of tube arcing, and the deep learning neural network 502 can have detected or recognized within the medical image 106 more granular visual evidence that suggests that such tube arcing is caused by a faulty insulator (e.g., as opposed to a fault vacuum enclosure). An insulator can malfunction with differing levels of severity. As the malfunctioning severity of the insulator increases, the remaining useful life of the insulator can commensurately decrease. Each unique level of malfunctioning severity, and thus each unique remaining useful life, of the insulator can be considered as leaving its own unique visual fingerprint or signature in the medical image 106. For instance, a mildly malfunctioning insulator (e.g., an insulator that is just beginning to degrade, thin, or corrode) can cause the medical image 106 to have a visual fingerprint/signature K and can be considered as having a lengthy remaining useful life. So, the visual fingerprint/signature K can be considered as being mapped to or otherwise indicative of the lengthy remaining useful life. In contrast, a severely malfunctioning insulator (e.g., an insulator that is significantly degraded, thinned, or corroded) can cause the medical image 106 to have a visual fingerprint/signature L and can be considered as having a short remaining useful life. So, the visual fingerprint/signature L can be considered as being mapped to or otherwise indicative of the short remaining useful life.

In any case, the deep learning neural network 702 can, due to its training described later herein, be able to detect or recognize whatever unique visual fingerprints or signatures are within the medical image 106 as supplemented by the failure classification label 304 and by the root cause classification label 504, and the deep learning neural network 702 can accordingly infer or predict the RUL 704.

As above, note that an accuracy level of the deep learning neural network 702 can be considered as being increased or heightened, due to the fact that the deep learning neural network 702 can be conditioned on (e.g., can receive as input) the failure classification label 304 and the root cause classification label 504. In other words, because the deep learning neural network 702 can receive as input the outputs produced by the deep learning neural network 302 and the deep learning neural network 502, the inferencing work performed by the deep learning neural network 302 and by the deep learning neural network 502 can be considered as restricting or shrinking the inferencing space or search space of the deep learning neural network 702. In still other words, the inferencing work performed by the deep learning neural network 302 and by the deep learning neural network 502 can be considered as helping, assisting, or otherwise supplementing the deep learning neural network 702 in the performance of its own inferencing work.

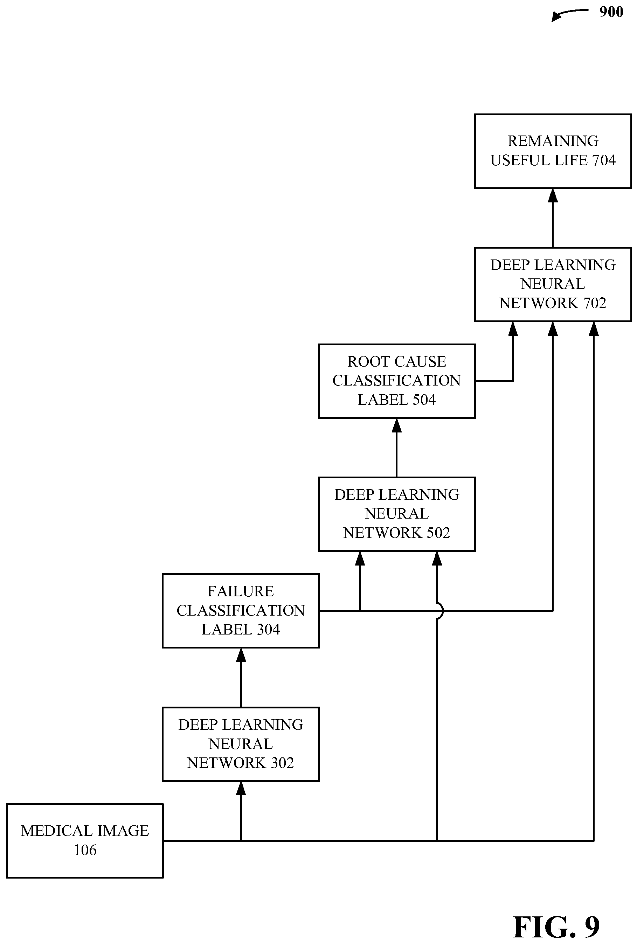

FIG. 9 illustrates an example, non-limiting block diagram 900 showing a cascaded deep learning pipeline for predicting medical imaging scanner faults in accordance with one or more embodiments described herein. That is, FIG. 9 illustrates how the deep learning neural network 302, the deep learning neural network 502, and the deep learning neural network 702 can be arranged or organized with respect to each other.

As shown, the deep learning neural network 302, the deep learning neural network 502, and the deep learning neural network 702 can be considered as being oriented or arranged in a cascaded layout. In such cascaded layout, the medical image 106 can be received as input by all three of the deep learning neural network 302, the deep learning neural network 502, and the deep learning neural network 702; the failure classification label 304 can be received as input by the deep learning neural network 502 and the deep learning neural network 702; and the root cause classification label 504 can be received as input by the deep learning neural network 702. In other words, this cascaded layout can be considered as first performing unconditioned failure classification on the medical image 106; as next performing root cause classification on the medical image 106 conditioned on the failure classification label 304; and then as performing remaining useful life regression or estimation on the medical image 106 conditioned on both the failure classification label 304 and the root cause classification label 504. Such cascaded layout can be considered as an interpretable or explainable pipeline for accurately or rigorously predicting or characterizing faults of the medical imaging scanner 104.

However, it should be appreciated that such cascaded layout is a mere non-limiting example. In various cases, any other suitable layout can be implemented.

As a non-limiting example, the deep learning neural network 302, the deep learning neural network 502, and the deep learning neural network 702 can, in some cases, be replaced with a single, overarching deep learning neural network (not shown) which can be trained or configured to receive as input the medical image 106 and to simultaneously produce as output all three of the failure classification label 304, the root cause classification label 504, and the RUL 704. In such case, the root cause classification label 504 and the RUL 704 can be ignored or discarded, in response to the failure classification label 304 indicating that no failure mode is detected in or evident from the medical image 106.

As another non-limiting example, the deep learning neural network 502 can be configured to receive as input only the medical image 106 (e.g., cannot receive as input the failure classification label 304), and the deep learning neural network 702 can likewise be configured to receive as input only the medical image 106 (e.g., cannot receive as input the failure classification label 304 nor the root cause classification label 504). In such situations, the deep learning neural network 502 can be executed on the medical image 106 to produce the root cause classification label 504, in response to the failure classification label 304 indicating that the medical imaging scanner 104 is experiencing some failure mode. Moreover, in such situations, there can be a plurality of RUL estimation neural networks (not shown) that can respectively correspond to the plurality of hardware components 202. That is, each of the plurality of RUL estimation neural networks can be configured or trained to receive as input a medical image and to produce as output an RUL estimation for a respective one of the plurality of hardware components 202 (e.g., there can be a first RUL estimation neural network that is configured to estimate remaining useful life for the hardware component 202(1); there can be an r-th RUL estimation neural network that is configured to estimate remaining useful life for the hardware component 202(*r*))*.* In such cases, the deep learning neural network 702 can be whichever of the plurality of RUL estimation neural networks that corresponds to whichever hardware component is indicated by the root cause classification label 504. However, in other cases, there can be no plurality of RUL estimation neural networks. Instead, the deep learning neural network 702 can be the only RUL estimation neural network, but it can be configured to estimate a remaining useful life for the entirety of the medical imaging scanner 104, rather than for any specific or individual hardware component of the medical imaging scanner 104.

As yet another non-limiting example, the deep learning neural network 502 can, in some embodiments, be excluded or omitted, and the deep learning neural network 702 can be configured to receive as input only the medical image 106. In such case, there can be no root cause classification label 504. Instead, the deep learning neural network 302 can produce the failure classification label 304 based on the medical image 106; and, in response to the failure classification label 304 indicating that the medical imaging scanner 104 is experiencing a failure mode, the deep learning neural network 702 can then be executed on the medical image 106 (e.g., receiving neither the failure classification label 304 nor the root cause classification label 504 as input) to produce as output the RUL 704. In such cases, the deep learning neural network 702 can be considered as estimating remaining useful life for the medical imaging scanner 104 as a whole, rather than for any specific or individual hardware component of the medical imaging scanner 104. This can nevertheless assist a technician associated with the medical imaging scanner 104, since whatever failure mode is indicated by the failure classification label 304 can be considered as conveying to the technician which general or coarse subset of the plurality of hardware components 202 warrants service. For instance, if the failure classification label 304 indicates that the medical imaging scanner 104 is suffering from a tube arcing failure mode, the technician can know or understand that an X-ray tube of the medical imaging scanner 104 should be serviced before the RUL 704 elapses. As another instance, if the failure classification label 304 indicates that the medical imaging scanner 104 is suffering from an optical obfuscation failure mode, the technician can know or understand that a detector or optical lens of the medical imaging scanner 104 should be serviced or cleaned before the RUL 704 elapses.

As even another non-limiting example, the deep learning neural network 302 can, in some embodiments, be excluded or omitted, and the deep learning neural network 702 can be configured to receive as input only the medical image 106. In such case, there can be no failure mode classification label 304. Instead, the deep learning neural network 502 can produce the root cause classification label 504 based on the medical image 106, where the root cause classification label 504 can include an (r + 1)-th class or probability score indicating the likelihood that none of the plurality of hardware components 202 is malfunctioning. So, in response to the root cause classification label 504 indicating that at least one of the plurality of hardware components 202 is not functioning appropriately, the deep learning neural network 702 can be executed on the medical image 106 (e.g., receiving neither the failure classification label 304 nor the root cause classification label 504 as input) to produce as output the RUL 704. As above, the deep learning neural network 702 can, in some of such cases, be the only RUL estimation neural network, such that the RUL 704 can be considered as indicating the remaining useful life for the medical imaging scanner 104 as a whole, rather than for any specific or individual hardware component of the medical imaging scanner 104. However, in others of such cases, there can instead be a plurality of RUL estimation neural networks respectively corresponding to the plurality of hardware components 202, and the deep learning neural network 702 can be whichever of the plurality of RUL estimation neural networks corresponds to whichever hardware component is indicated by the root cause classification label 504.

As yet another non-limiting example, the deep learning neural network 702 can, in various embodiments, be executed on the medical image 106 before execution of the deep learning neural network 302 or of the deep learning neural network 502. In such case, the RUL 704 can be considered as indicating the remaining useful life of the medical imaging scanner 104 as a whole, and the deep learning neural network 302 or the deep learning neural network 502 can be executed on the medical image 106 in response to the RUL 704 failing to satisfy any suitable threshold. In other words, there can be no need for failure mode classification or root cause classification if the RUL 704 indicates that the medical imaging scanner 104 has plenty of useful life remaining. In contrast, if the RUL 704 instead indicates that the medical imaging scanner 104 has little useful life remaining, then failure mode classification or root cause classification can be warranted.

As still another non-limiting example, the deep learning neural network 302 and the deep learning neural network 502 can, in various embodiments, both be excluded or omitted. Moreover, rather than having a single instantiation of the deep learning neural network 702, there can be r instantiations of the deep learning neural network 702, each being configured or trained to perform RUL estimation for a respective one of the plurality of hardware component 202. In such cases, each of those r instantiations of the deep learning neural network 702 can be executed independently or in parallel on the medical image 106, thereby yielding a total of *r* RULs, each of which can indicate a remaining useful life of a respective one of the plurality of hardware components 202 (e.g., a first RUL for the hardware component 202(1); an r-th RUL for the hardware component 202(*r*))*.* Accordingly, if a given RUL of those *r* RULs fails to satisfy any suitable threshold, it can be concluded that whichever hardware component corresponds to that given RUL is malfunctioning and thus warrants or merits servicing prior to that given RUL elapsing.

No matter what specific deep learning pipeline or arrangement is implemented by various embodiments described herein, all of such embodiments can be considered as leveraging deep learning so as to infer or predict failure information regarding the medical imaging scanner 104, based on the medical image 106 (e.g., such failure information can be visually manifested as unique artifacts or distortions in the medical image 106, and such unique artifacts or distortions can be recognized by deep learning). Such prediction can be facilitated, even if the medical imaging scanner 104 lacks or is otherwise not equipped with internal operability sensors.

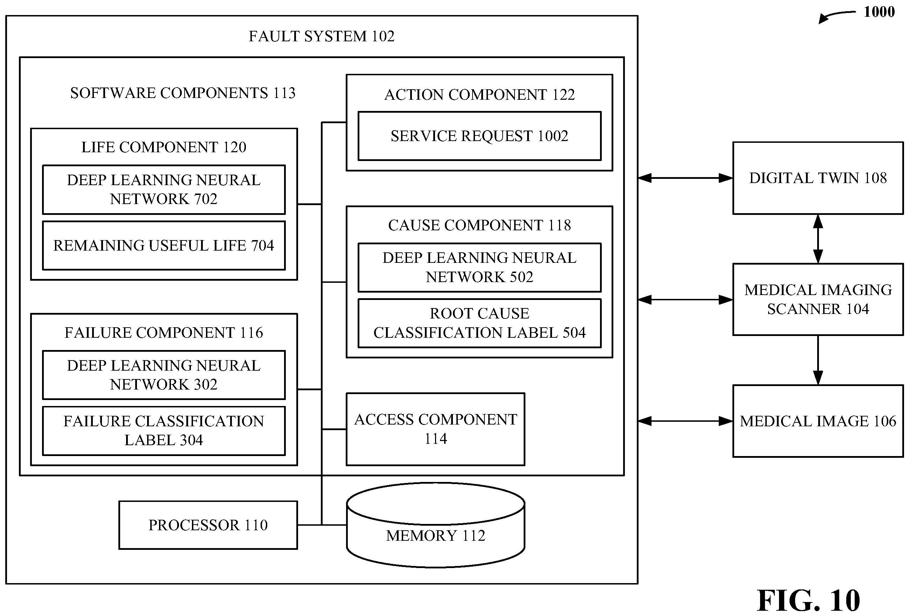

In various embodiments, the action component 122 can electronically perform or initiate any suitable electronic actions, based on the generation of the failure classification label 304, of the root cause classification label 504, or of the RUL 704. Non-limiting aspects are described with respect to FIGs. 10-16.

FIG. 10 illustrates a block diagram of an example, non-limiting system 1000 including a service request that can facilitate scanner fault prediction via image-based deep learning in accordance with one or more embodiments described herein. As shown, the system 1000 can, in some cases, comprise the same components as the system 700, and can further comprise a service request 1002.

In various embodiments, the action component 122 can, in response to the failure classification label 304 indicating that the medical imaging scanner 104 is afflicted with at least one of the plurality of defined scanning failure modes 402, electronically transmit the service request 1002 to any suitable computing device associated with a technician of the medical imaging scanner 104. In some instances, the action component 122 can electronically transmit the service request 1002 to the medical imaging scanner 104 itself, so as to be visually or audibly presented or displayed by any suitable computer screens or computer speakers of the medical imaging scanner 104. In any case, the service request 1002 can be any suitable electronic message that instructs, commands, asks, or notifies that the medical imaging scanner 104 be serviced so as to remedy whatever defined scanning failure mode is indicated by the failure classification label 304.

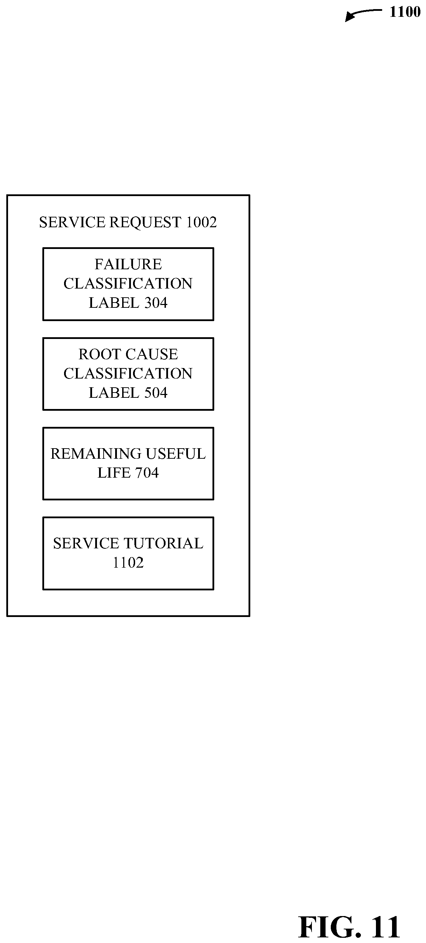

FIG. 11 illustrates an example, non-limiting block diagram 1100 showing the service request 1002 in accordance with one or more embodiments described herein.

As shown, the service request 1002 can, in some aspects, comprise, contain, or otherwise include the failure classification label 304 and the root cause classification label 504. Accordingly, the service request 1002 can be interpreted as indicating or notifying the technician that whichever defined scanning failure mode that is indicated by the failure classification label 304 can be remedied or rectified by repairing or replacing whichever hardware component that is indicated by the root cause classification label 504. In this way, the service request 1002 can be considered as pointing out for the technician which specific or granular component of the medical imaging scanner 104 should be serviced, thereby assisting the technician.

As also shown, the service request 1002 can, in some instances, comprise, contain, or otherwise include the RUL 704. Accordingly, the service request 1002 can be interpreted as indicating or notifying the technician of a timeline according to which or in compliance with which the hardware component that is indicated by the root cause classification label 504 should be repaired or replaced. As a non-limiting example, the action component 122 can identify a current or present time/date (e.g., via any suitable electronic clock or calendar), and the action component 122 can identify a future time/date that is equal to or otherwise based on adding the RUL 704 to that current or present time/date. In other words, that future time/date can be considered as the time/date at which the RUL 704 will or otherwise is expected to elapse. In such case, the service request 1002 can be considered as instructing, commanding, or notifying the technician that the hardware component indicated by the root cause classification label 504 should be repaired or replaced prior to (e.g., no later than) the future time/date, thereby assisting the technician.

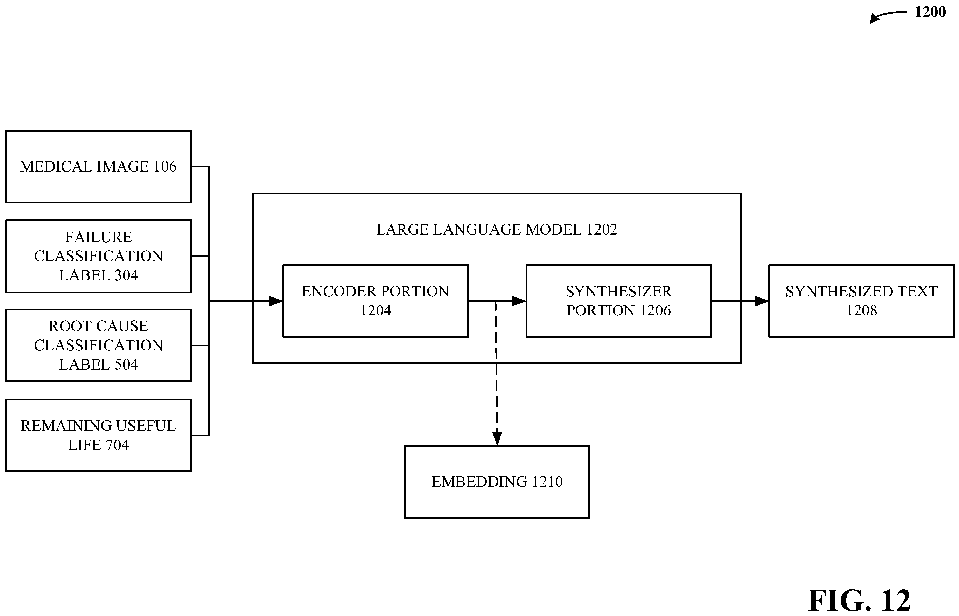

As also shown, the service request 1002 can, in some aspects, comprise, contain, or otherwise include a service tutorial 1102. In various instances, the service tutorial 1102 can be any suitable electronic data that explains, describes, or otherwise teaches how the hardware component that is indicated by the root cause classification label 504 should be repaired or replaced, so as to address or rectify the defined scanning failure mode indicated by the failure classification label 304. Non-limiting aspects are described with respect to FIGs. 12-14.

FIGs. 12-14 illustrate example, non-limiting block diagrams 1200, 1300, and 1400 showing how the service tutorial 1102 can be generated in accordance with one or more embodiments described herein.

First, consider FIG. 12. In various embodiments, there can be a large language model 1202 (hereafter "LLM 1202"). In various aspects, the LLM 1202 can comprise an encoder portion 1204 and a synthesizer portion 1206. In various cases, the encoder portion 1204 can be considered as being upstream from the synthesizer portion 1206. Equivalently, the synthesizer portion 1206 can be considered as being downstream of the encoder portion 1204.

In various aspects, the encoder portion 1204 can exhibit any suitable deep learning internal architecture. Indeed, in various cases, the encoder portion 1204 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

Likewise, in various instances, the synthesizer portion 1206 can exhibit any suitable deep learning internal architecture. Indeed, in various cases, the synthesizer portion 1206 can have an input layer, one or more hidden layers, and an output layer. In various instances, any of such layers can be coupled together by any suitable interneuron connections or interlayer connections (e.g., forward connections, skip connections, recurrent connections). Furthermore, in various cases, any of such layers can be any suitable types of neural network layers having any suitable learnable or trainable internal parameters (e.g., any of such input layer, one or more hidden layers, or output layer can be convolutional layers, dense layers, batch normalization layers, LSTM layers, or transformer layers). Further still, in various cases, any of such layers can be any suitable types of neural network layers having any suitable fixed or non-trainable internal parameters (e.g., any of such input layer, one or more hidden layers, or output layer can be non-linearity layers, padding layers, pooling layers, or concatenation layers).

Regardless of the specific internal architecture that is implemented within the encoder portion 1204, the encoder portion 1204 can be configured to receive textual data (which can be accompanied by any suitable numerical or graphical data) and to produce embeddings based on such inputted textual data. In contrast, regardless of the specific internal architecture that is implemented within the synthesizer portion 1206, the synthesizer portion 1206 can be configured to receive embeddings produced by the encoder portion 1204 and to produce synthesized textual content based on such embeddings. As some non-limiting examples, the LLM 1202 can be any of the following: ChatGPT; Gene.AI ^{®}; Ollama ^{®}; Bard ^{®}; Claude ^{®}; Seamless ^{®}; GitHub CoPilot ^{®}; or Amazon CodeWhisperer ^{®}.

In various aspects, the action component 122 can electronically execute the LLM 1202 on the medical image 106, on the failure classification label 304, on the root cause classification label 504, or on the RUL 704. In various instances, such execution can cause the LLM 1202 to produce some synthesized text 1208.

More specifically, the action component 122 can concatenate the medical image 106, the failure classification label 304, the root cause classification label 504, and the RUL 704 (or any combination thereof) together. In various aspects, the action component 122 can feed or route that concatenation to the input layer of the encoder portion 1204, that concatenation can complete a forward pass through the one or more hidden layers of the encoder portion 1204, and an output layer of the encoder portion 1204 can compute or otherwise calculate an embedding 1210, based on activation maps or feature maps provided by the one or more hidden layers of the encoder portion 1204.

In various instances, the embedding 1210 can be considered as a latent vector representation that the encoder portion 1204 believes or infers corresponds to the medical image 106, the failure classification label 304, the root cause classification label 504, and the RUL 704. More specifically, the embedding 1210 can be one or more scalars, one or more vectors, one or more matrices, one or more tensors, or any suitable combination thereof. In various aspects, the dimensionality of the embedding 1210 (e.g., the total number or cardinality of numerical elements within the embedding 1210) can be smaller (e.g., many orders of magnitude smaller, in some cases) than the collective dimensionality of the medical image 106, the failure classification label 304, the root cause classification label 504, and the RUL 704. In various instances, despite its smaller dimensionality, the embedding 1210 can nevertheless be considered as representing, albeit in hidden or non-apparent fashion, at least some substantive or semantic content of the medical image 106, the failure classification label 304, the root cause classification label 504, and the RUL 704. In other words, the embedding 1210 can be considered as a compact or compressed numerical representation of the medical image 106, the failure classification label 304, the root cause classification label 504, and the RUL 704. Note that the embedding 1210 can be considered as representing the medical image 106, the failure classification label 304, the root cause classification label 504, and the RUL 704 in a latent, obscure, or otherwise hidden fashion, since a third-party that has no connection or relationship to the encoder portion 1204 would be unable to recreate or guess the medical image 106, the failure classification label 304, the root cause classification label 504, and the RUL 704 from the embedding 1210 alone.

Now, in various aspects, the embedding 1210 can be fed or routed to the input layer of the synthesizer portion 1206, the embedding 1210 can complete a forward pass the through one or more hidden layers of the synthesizer portion 1206, and the output layer of the synthesizer portion 1206 can compute or otherwise calculate the synthesized text 1208, based on activation maps or feature maps provided by the one or more hidden layers of the synthesizer portion 1206.

In various aspects, the synthesized text 1208 can be one or more declarative sentences or sentence fragments that the synthesizer portion 1206 has generated based on the embedding 1210. Note that the synthesized text 1208 can be not an estimated or approximated reconstruction of the medical image 106, the failure classification label 304, the root cause classification label 504, and the RUL 704. Instead, the synthesized text 1208 can be any suitable number of synthetic sentences that somehow semantically or substantively relate to the embedding 1210 and thus to the medical image 106, the failure classification label 304, the root cause classification label 504, and the RUL 704. In some cases, the synthesized text 1208 can be considered as containing hallucinations that are semantically or substantively related to the medical image 106, the failure classification label 304, the root cause classification label 504, and the RUL 704.

In various aspects, the action component 122 can ignore, discard, or delete the synthesized text 1208. However, the action component 122 can record, preserve, store, or otherwise maintain the embedding 1210. In other words, the action component 122 can extract the embedding 1210 from the encoder portion 1204 (e.g., from hidden layers of the LLM 1202).

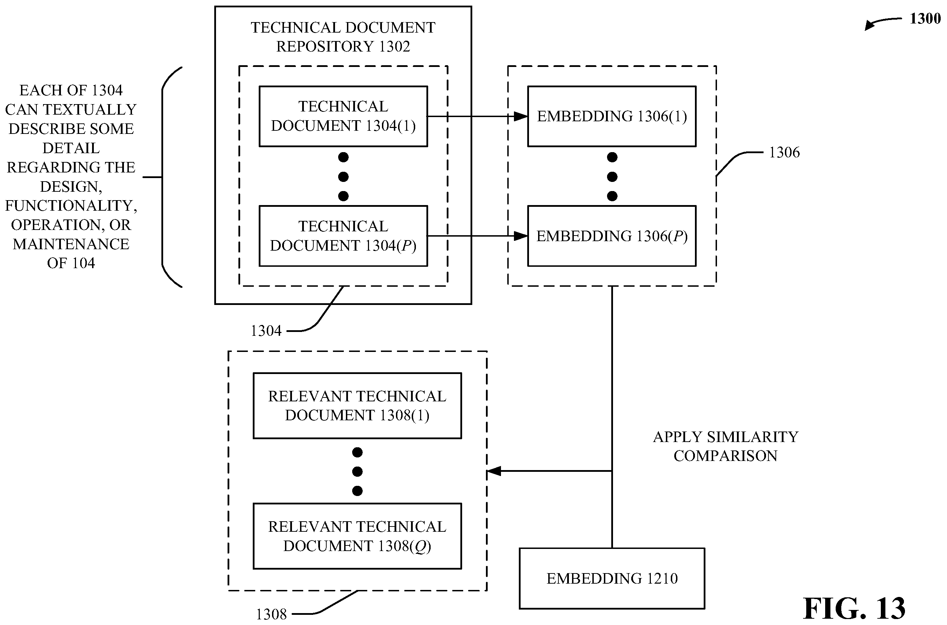

Now, consider FIG. 13. In various embodiments, there can be a technical document repository 1302. In various aspects, the technical document repository 1302 can comprise a plurality of technical documents 1304. In various instances, the plurality of technical documents 1304 can comprise *p* documents, for any suitable positive integer *p* > 1: a technical document 1304(1) to a technical document 1304(*p*). In various instances, each of the plurality of technical documents 1304 can be any suitable electronic file (e.g., word-doc file, portable document format (PDF) file, webpage file) that textually (or, in some cases, graphically or numerically) describes, teaches, shows, indicates, or otherwise conveys one or more technical details, teachings, characteristics, or other aspects (e.g., how to operate, how to maintain, how to service, how to troubleshoot) of the medical imaging scanner 104 or of any of the plurality of hardware components 202. As some non-limiting examples, any of the plurality of technical documents 1304 can be a sentence, a paragraph, a page, a section, a chapter, or an entirety of any of the following: a service manual or handbook of the medical imaging scanner 104 or of some respective hardware component thereof, a bill of materials of the medical imaging scanner 104 or of some respective hardware component thereof, a blueprint or schematic of the medical imaging scanner 104 or of some respective hardware component thereof, or a failure mode analysis report of the medical imaging scanner 104 or of some respective hardware component thereof.

It is to be appreciated that the technical document repository 1302 need not be centralized. In other words, the technical document repository 1302 can be made up of any suitable number of decentralized shards or sub-repositories.

Now, in various aspects, the action component 122 can electronically identify a set of relevant technical documents 1308 within the technical document repository 1302. In particular, the action component 122 can facilitate such identification via embedding-based searching. Indeed, the action component 122 can generate a plurality of embeddings 1306, by executing the LLM 1202 on each of the plurality of technical documents 1304 and extracting from the encoder portion 1204 a respective embedding.

As a non-limiting example, the action component 122 can execute the LLM 1202 on the technical document 1304(1), and the action component 122 can extract, during that execution, an embedding 1306(1). Indeed, the action component 122 can feed or route the technical document 1304(1) to the input layer of the encoder portion 1204, the technical document 1304(1) can complete a forward pass through the one or more hidden layers of the encoder portion 1204, and the output layer of the encoder portion 1204 can compute or otherwise calculate the embedding 1306(1), based on activation maps or feature maps provided by the one or more hidden layers of the encoder portion 1204. So, the embedding 1306(1) can be considered as a latent vector representation of the technical document 1304(1). In some cases, the embedding 1306(1) can then complete a forward pass through the synthesizer portion 1206, but the action component 122 can ignore, disregard, or delete whatever synthesized textual content the synthesizer portion 1206 creates based on the embedding 1306(1).

As another non-limiting example, the action component 122 can execute the LLM 1202 on the technical document 1304(p), and the action component 122 can extract, during that execution, an embedding 1306(*p*). In particular, the action component 122 can feed or route the technical document 1304(*p*) to the input layer of the encoder portion 1204, the technical document 1304(*p*) can complete a forward pass through the one or more hidden layers of the encoder portion 1204, and the output layer of the encoder portion 1204 can compute or otherwise calculate the embedding 1306(*p*), based on activation maps or feature maps provided by the one or more hidden layers of the encoder portion 1204. Thus, the embedding 1306(*p*) can be considered as a latent vector representation of the technical document 1304(*p*). As above, the embedding 1306(*p*) can then complete a forward pass through the synthesizer portion 1206, but the action component 122 can ignore, disregard, or delete whatever synthesized textual content the synthesizer portion 1206 creates based on the embedding 1306(*p*).

Note that the embedding 1306(1) to the embedding 1306(p) can collectively be considered as the plurality of embeddings 1306. In various aspects, the action component 122 can electronically identify the set of relevant technical documents 1308 by comparing the embedding 1210 with the plurality of embeddings 1306. Indeed, for each given embedding of the plurality of embeddings 1306, the action component 122 can compute any suitable error or similarity value (e.g., mean absolute error (MAE), mean squared error (MSE), cosine similarity, Euclidean distance, cross-entropy) between that given embedding and the embedding 1210. Accordingly, the action component 122 can identify as the set of relevant technical documents 1308 whichever of the plurality of technical documents 1304 whose embeddings are most similar to, closest to, or otherwise within any suitable threshold margin of the embedding 1210. In the non-limiting example of FIG. 13, the set of relevant technical documents 1308 can comprise *q* documents, for any suitable positive integer *q* < *p*: a relevant technical document 1308(1) to a relevant technical document 1308(*q*).

In any case, the set of relevant technical documents 1308 can be considered as being whichever of the plurality of technical documents 1304 that are semantically or substantively related to the medical image 106, to the failure classification label 304, to the root cause classification label 504, and to the RUL 704. That is, the set of relevant technical documents 1308 can be considered as being whichever of the plurality of technical documents 1304 contain information that pertains to: remedying the defined scanning failure mode indicated by the failure classification label 304; or servicing or maintenance of the hardware component indicated by the root cause classification label 504.

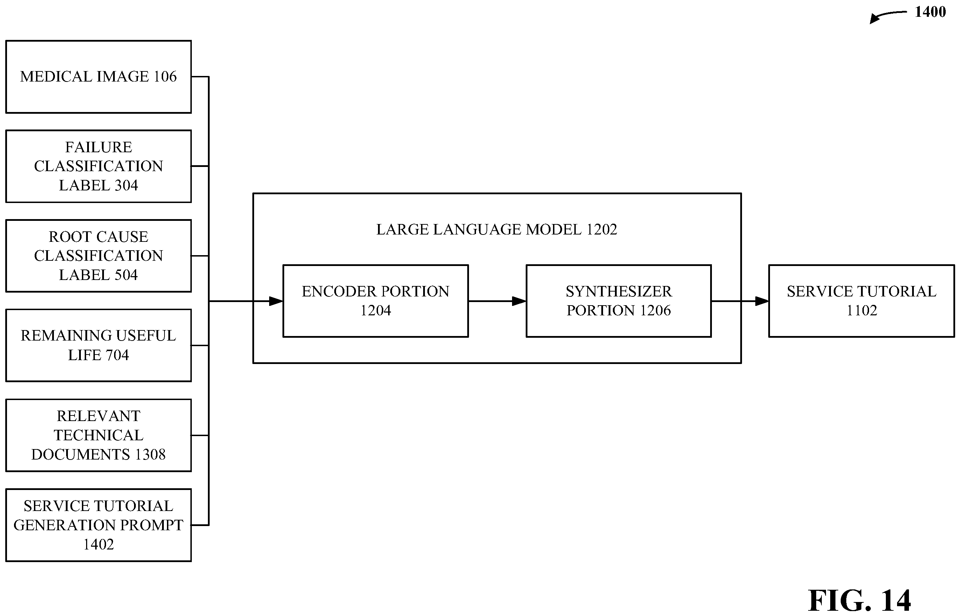

Now, consider FIG. 14. In various embodiments, the action component 122 can electronically execute the LLM 1202 on the medical image 106, on the failure classification label 304, on the root cause classification label 504, on the RUL 704, and on the set of relevant technical documents 1308. In various instances, such execution can cause the LLM 1202 to produce the service tutorial 1102.

More specifically, there can be a service tutorial generation prompt 1402. In various aspects, the service tutorial generation prompt 1402 can be one or more unstructured or plain text sentences or sentence fragments that request or command identification of how whatever hardware component that is indicated in the root cause classification label 504 and having the RUL 704 can or should be repaired or replaced so as to solve the defined scanning failure mode indicated in the failure classification label 304. As a non-limiting example, the service tutorial generation prompt 1402 can be the following sentences: "Problem defined by the medical image 106, the failure classification label 304, the root cause classification label 504, and the RUL 704. Using the set of relevant technical documents 1308, describe the instructions for solving the problem.". As another non-limiting example, the service tutorial generation prompt 1402 can be the following sentence: "Problem defined by the medical image 106, the failure classification label 304, the root cause classification label 504, and the RUL 704. According to the set of relevant technical documents 1308, what are the steps for solving the problem?".

In various aspects, the action component 122 can concatenate the medical image 106, the failure classification label 304, the root cause classification label 504, the RUL 704, and the set of relevant technical documents 1308 (or any combination thereof) together with the service tutorial generation prompt 1402. In various aspects, the action component 122 can feed or route that concatenation to the input layer of the encoder portion 1204, that concatenation can complete a forward pass through the one or more hidden layers of the encoder portion 1204, and the output layer of the encoder portion 1204 can compute or otherwise calculate one or more embeddings (not shown), based on activation maps or feature maps provided by the one or more hidden layers of the encoder portion 1204. In various cases, those one or more embeddings can be routed to the input layer of the synthesizer portion 1206, those one or more embeddings can complete a forward pass through the one or more hidden layers of the synthesizer portion 1206, and the output layer of the synthesizer portion 1206 can compute or otherwise calculate the service tutorial 1102 based on activation maps or feature maps provided by the one or more hidden layers of the synthesizer portion 1206. In any case, the service tutorial 1102 can be one or more unstructured or plain text declarative sentences or sentence fragments that semantically answer or respond to the service tutorial generation prompt 1402. That is, the service tutorial 1102 can be synthesized text that describes, explains, shows, or otherwise teaches what specific sequence of steps should (as inferred by the LLM 1202 based on the content of the set of relevant technical documents 1308) be taken to repair, replace, or otherwise service the hardware component indicated in the root cause classification label 504 so as to solve the defined scanning failure mode indicated in the failure classification label 304. In some cases, the service tutorial 1102 can comprise synthesized diagrams or images that help to elaborate or elucidate such sequence of steps.

Accordingly, since the service tutorial 1102 can be included in the service request 1002, the service request 1002 can be considered as notifying the technician of the medical imaging scanner 104 of specific servicing actions that should be taken to address the defined scanning failure mode indicated in the failure classification label 304, thereby further assisting the technician.

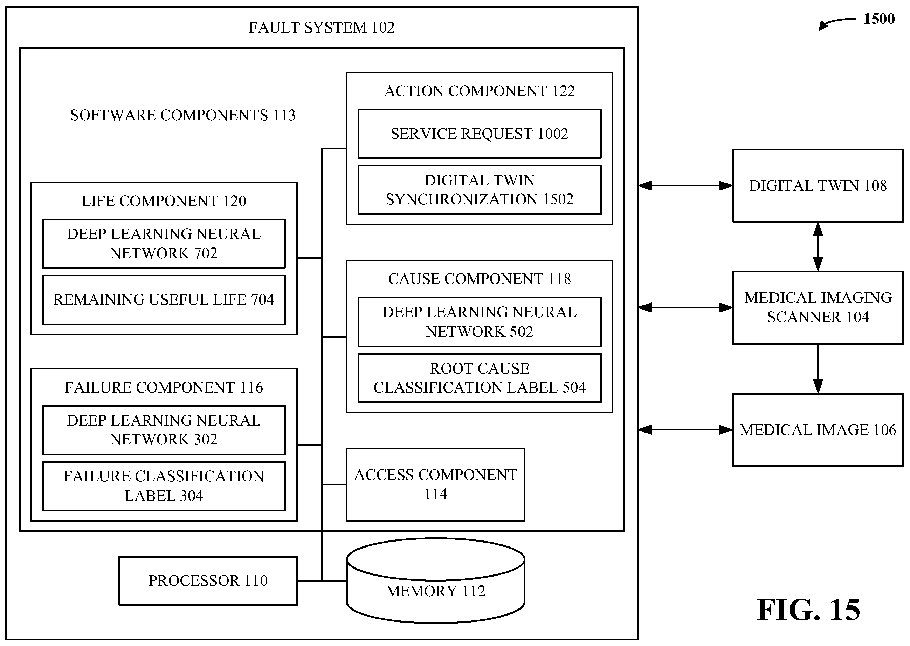

FIG. 15 illustrates a block diagram of an example, non-limiting system 1500 including a digital twin synchronization that can facilitate scanner fault prediction via image-based deep learning in accordance with one or more embodiments described herein. As shown, the system 1500 can, in some cases, comprise the same components as the system 1000, and can further comprise a digital twin synchronization 1502.

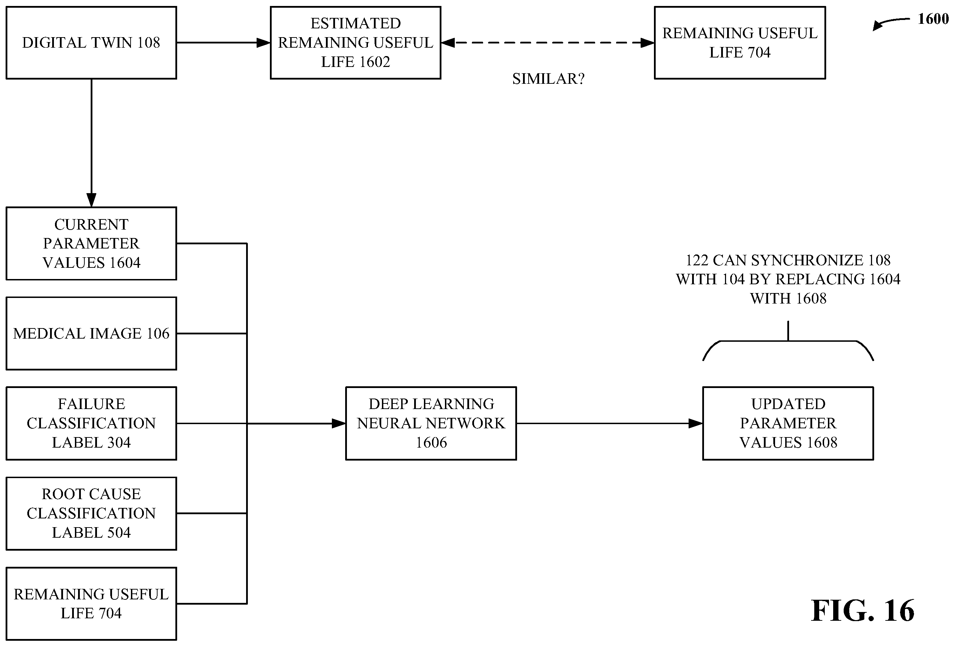

In various embodiments, the digital twin synchronization 1502 can be an electronic action that updates parametric state values of the digital twin 108. In various aspects, the action component 122 can electronically perform the digital twin synchronization 1502 on the digital twin 108, based on the RUL 704. Non-limiting aspects are described with respect to FIG. 16.

FIG. 16 illustrates an example, non-limiting block diagram 1600 showing how the digital twin 108 can be synchronized to the medical imaging scanner 104 in accordance with one or more embodiments described herein.

In various aspects, as mentioned above, the digital twin 108 can predict, forecast, or simulate any suitable behavior of the medical imaging scanner 104. It can be possible for the values that are assigned to the parametric state 204 do not match the actual parametric values that are exhibited by the medical imaging scanner 104 in reality (e.g., the true parametric state of the medical imaging scanner 104 can drift over time or with use), which can render the predictions, forecasts, or simulations of the digital twin 108 incorrect. The digital twin 108 can, in such case, be considered as not being synchronized with the medical imaging scanner 104. In various instances, the action component 122 can electronically determine, based on the RUL 704, whether or not the digital twin 108 is synchronized with the medical imaging scanner 104. If the action component 122 determines that the digital twin 108 is not synchronized with the medical imaging scanner 104, then the action component 122 can perform the digital twin synchronization 1502.

More specifically, the digital twin 108 can predict, forecast, or simulate an estimated remaining useful life 1602 (hereafter "estimated RUL 1602") for the hardware component that is indicated in the root cause classification label 504. Indeed, the estimated RUL 1602 can be a specific value of a respective one of the set of output variables 208. In various aspects, the action component 122 can compute a difference or error between the RUL 704 and the estimated RUL 1602. If that difference or error satisfies any suitable threshold, the remaining useful life computation performed by the digital twin 108 can be considered as being consistent with that of the deep learning neural network 702, and the action component 122 can accordingly conclude that the digital twin 108 is synchronized with the medical imaging scanner 104. However, if that difference or error fails to satisfy the threshold, the remaining useful life computation performed by the digital twin 108 can be considered as being inconsistent with that of the deep learning neural network 702, and the action component 122 can accordingly conclude that the digital twin 108 is not synchronized with the medical imaging scanner 104.

In response to determining that the digital twin 108 is not synchronized with the medical imaging scanner 104, the action component 122 can perform the digital twin synchronization 1502, by leveraging a set of current parameter values 1604 of the digital twin 108 and by leveraging a deep learning neural network 1606.

In various aspects, the set of current parameter values 1604 can be whatever values are currently or presently assigned to the parametric state 204 (e.g., the set of current parameter values 1604 can have a first value that is currently or presently assigned to the parameter 204(1); the set of current parameter values 1604 can have an s-th value that is currently or presently assigned to the parameter 204(s)).

In various aspects, the deep learning neural network 1606 can exhibit any suitable deep learning internal architecture. Indeed, in various cases, the deep learning neural network 1606 can have an input layer, one or more hidden layers, and an output layer. In various instances, any of such layers can be coupled together by any suitable interneuron connections or interlayer connections, such as forward connections, skip connections, or recurrent connections. Furthermore, in various cases, any of such layers can be any suitable types of neural network layers having any suitable learnable or trainable internal parameters. For example, any of such input layer, one or more hidden layers, or output layer can be convolutional layers, whose learnable or trainable parameters can be convolutional kernels. As another example, any of such input layer, one or more hidden layers, or output layer can be dense layers, whose learnable or trainable parameters can be weight matrices or bias values. As still another example, any of such input layer, one or more hidden layers, or output layer can be batch normalization layers, whose learnable or trainable parameters can be shift factors or scale factors. As even another example, any of such input layer, one or more hidden layers, or output layer can be LSTM layers, whose learnable or trainable parameters can be input-state weight matrices or hidden-state weight matrices. As yet another example, any of such input layer, one or more hidden layers, or output layer can be transformer layers, whose learnable or trainable parameters can be single-head or multi-head attention blocks or other weight matrices. Further still, in various cases, any of such layers can be any suitable types of neural network layers having any suitable fixed or non-trainable internal parameters. For example, any of such input layer, one or more hidden layers, or output layer can be non-linearity layers, padding layers, pooling layers, or concatenation layers.

Regardless of its specific internal architecture, the deep learning neural network 1606 can be configured as a digital twin parameter predictor that is conditioned on the medical image 106 and on the outputs of the deep learning neural network 302, of the deep learning neural network 502, or of the deep learning neural network 702.

In various aspects, the action component 122 can electronically execute the deep learning neural network 1606 on the set of current parameter values 1604, on the medical image 106, on the failure classification label 304, on the root cause classification label 504, or on the RUL 704, and such execution can yield a set of updated parameter values 1608. More specifically, the action component 122 can concatenate the set of current parameter values 1604, the medical image 106, the failure classification label 304, the root cause classification label 504, and the RUL 704 (or any combination thereof) together. In various instances, the action component 122 can feed that concatenation to the input layer of the deep learning neural network 1606. In various cases, that concatenation can complete a forward pass through the one or more hidden layers of the deep learning neural network 1606. In various aspects, the output layer of the deep learning neural network 1606 can compute or calculate the set of updated parameter values 1608 based on activation maps or feature maps produced by the one or more hidden layers of the deep learning neural network 1606. In various instances, the set of updated parameter values 1608 can be considered as new or different versions of the set of current parameter values 1604 that are modified, altered, or otherwise adjusted so as to reflect (in the opinion of the deep learning neural network 1606) the true parametric state of the medical imaging scanner 104 (e.g., the set of updated parameter values 1608 can have a first updated value that corresponds to the parameter 204(1); the set of updated parameter values 1608 can have an s-th updated value that corresponds to the parameter 204(s)). In various cases, the action component 122 can perform the digital twin synchronization 1502, by replacing or overwriting the set of current parameter values 1604 with the set of updated parameter values 1608 (e.g., by assigning to the parameter 204(1) the first updated value of the set of updated parameter values 1608; by assigning to the parameter 204(s) the s-th updated value of the set of updated parameter values 1608).

In this way, the digital twin 108 can be synchronized with the medical imaging scanner 104, notwithstanding that the medical imaging scanner 104 can lack internal operability sensors.

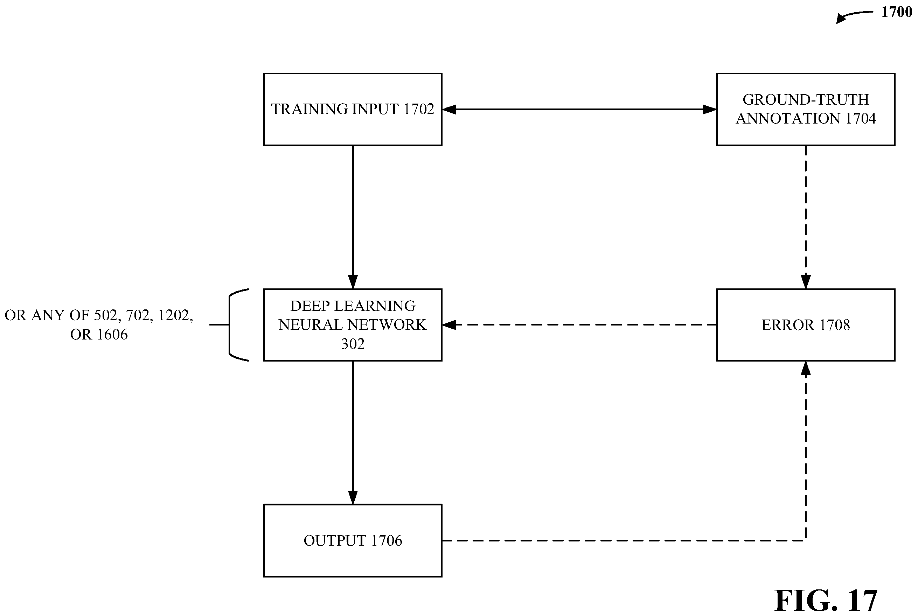

In order for the herein-described scanner fault predictions to be accurate, correct, or reliable, the various machine learning models described herein can first undergo training. A non-limiting example of such training is described with respect to FIG. 17.

FIG. 17 illustrates an example, non-limiting block diagram 1700 showing how various artificial intelligence models can be trained in accordance with one or more embodiments described herein.

In various aspects, prior to beginning training, the trainable internal parameters (e.g., convolutional kernels, weight matrices, bias values) of whatever artificial intelligence model is being trained (e.g., the deep learning neural network 302, the deep learning neural network 502, the deep learning neural network 702, the LLM 1202, the deep learning neural network 1606) can be initialized in any suitable fashion (e.g., via random initialization).

In various embodiments, there can be a training input 1702 and a ground-truth annotation 1704. When it is desired to train the deep learning neural network 302, the training input 1702 can be any suitable training medical image, and the ground-truth annotation 1704 can be whatever correct or accurate failure classification label is known or deemed to correspond to the training input 1702. When it is desired to train the deep learning neural network 502, the training input 1702 can be a concatenation of any suitable training medical image with any suitable training failure classification label, and the ground-truth annotation 1704 can be whatever correct or accurate root cause classification label is known or deemed to correspond to the training input 1702. When it is desired to train the deep learning neural network 702, the training input 1702 can be a concatenation of any suitable training medical image with any suitable training failure classification label and with any suitable root cause classification label, and the ground-truth annotation 1704 can be whatever correct or accurate remaining useful life is known or deemed to correspond to the training input 1702. When it is desired to train the LLM 1202, the training input 1702 can be any suitable training text, and the ground-truth annotation 1704 can be whatever correct or accurate synthesized textual content is known or deemed to correspond to the training input 1702. When it is desired to train the deep learning neural network 1606, the training input 1702 can be a concatenation of any suitable set of current digital twin parameter values, any suitable training medical image, any suitable training failure classification label, any suitable training root cause classification label, and any suitable training remaining useful life (or any other combination thereof), and the ground-truth annotation 1704 can be whatever correct or accurate set of updated digital twin parameter values is known or deemed to correspond to the training input 1702.

In any case, the artificial intelligence model that is being trained can be executed on the training input 1702, thereby causing that artificial intelligence model to produce an output 1706. More specifically, in some cases, the training input 1702 can be fed or routed to the input layer of the artificial intelligence model, the training input 1702 can complete a forward pass through the one or more hidden layers of the artificial intelligence model, and the output layer of the artificial intelligence model can compute the output 1706 based on activation maps or feature maps provided by the one or more hidden layers of the artificial intelligence model.

Note that the format, size, or dimensionality of the output 1706 can be dictated by the number, arrangement, sizes, or other characteristics of the neurons, convolutional kernels, LSTM layers, or other internal parameters of the output layer (or of any other layers) of the artificial intelligence model. Accordingly, the output 1706 can be forced to have any desired format, size, or dimensionality, by adding, removing, or otherwise adjusting characteristics of the output layer (or of any other layers) of the artificial intelligence model.

In various aspects, if the output 1706 is produced by the deep learning neural network 302, the output 1706 can be considered as the predicted or inferred failure classification label that the deep learning neural network 302 believes should correspond to the training input 1702. If the output 1706 is produced by the deep learning neural network 502, the output 1706 can be considered as the predicted or inferred root cause classification label that the deep learning neural network 502 believes should correspond to the training input 1702. If the output 1706 is produced by the deep learning neural network 702, the output 1706 can be considered as the predicted or inferred remaining useful life that the deep learning neural network 702 believes should correspond to the training input 1702. If the output 1706 is produced by the LLM 1202, the output 1706 can be considered as the predicted or inferred text (e.g., predicted or inferred service tutorial) that the LLM 1202 believes should correspond to the training input 1702. If the output 1706 is produced by the deep learning neural network 1606, the output 1706 can be considered as the predicted or inferred updated digital twin parameter values that the deep learning neural network 1606 believes should correspond to the training input 1702. Note that, if the artificial intelligence model that is being trained has so far undergone no or little training, then the output 1706 can be highly inaccurate. In other words, the output 1706 can be very different from the ground-truth annotation 1704.

In various aspects, an error 1708 (e.g., MAE, MSE, cross-entropy error) between the output 1706 and the ground-truth annotation 1704 can be computed. In various instances, the trainable internal parameters of the artificial intelligence model can be incrementally updated via backpropagation (e.g., stochastic gradient descent) based on the error 1708.

In various cases, such execution-and-update procedure can be repeated for any suitable number of input-annotation pairs. This can ultimately cause the trainable internal parameters of the artificial intelligence model (e.g., of the deep learning neural network 302, of the deep learning neural network 502, of the deep learning neural network 702, of the LLM 1202, of the deep learning neural network 1606) to become iteratively optimized for accurately performing its inferencing task (e.g., failure classification, root cause classification, RUL estimation, text synthesis, digital twin parameter estimation). In various aspects, any suitable training batch sizes, any suitable error/loss functions, or any suitable training termination criteria can be utilized during such training.

Although the herein disclosure mainly describes the various artificial intelligence models as being trained in supervised fashion, this is a mere non-limiting example for ease of explanation and illustration. In various embodiments, any other suitable training paradigms can be used to train the deep learning neural network 302, the deep learning neural network 502, the deep learning neural network 702, the LLM 1202, or the deep learning neural network 1606, such as unsupervised training or reinforcement learning, any of which may be federated or non-federated.

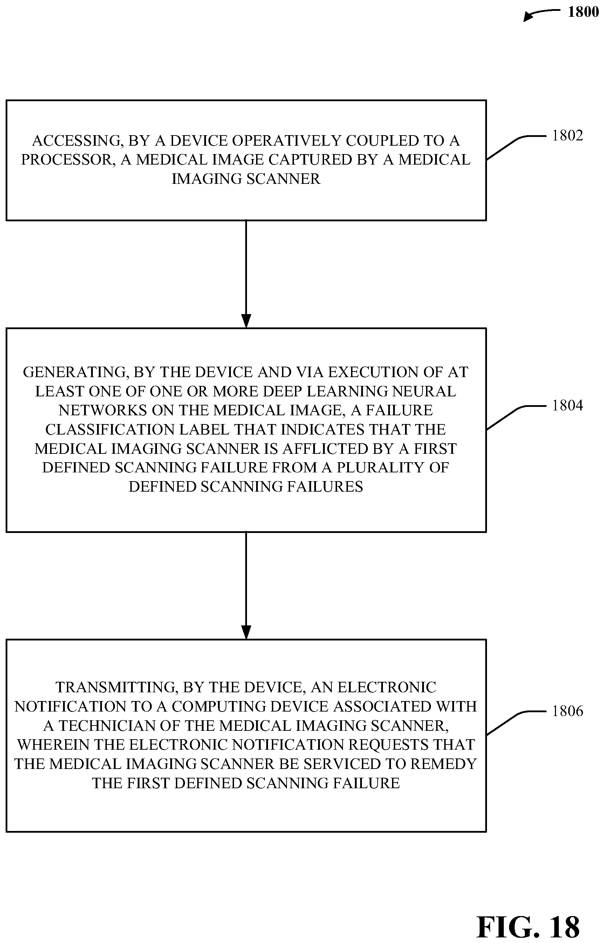

FIG. 18 illustrates a flow diagram of an example, non-limiting computer-implemented method 1800 that can facilitate scanner fault prediction via image-based deep learning in accordance with one or more embodiments described herein. In various cases, the fault system 102 can facilitate the computer-implemented method 1800.

In various embodiments, act 1802 can include accessing, by a device (e.g., via 114) operatively coupled to a processor (e.g., 110), a medical image (e.g., 106) captured by a medical imaging scanner (e.g., 104).

In various aspects, act 1804 can include generating, by the device (e.g., via 116) and via execution of at least one of one or more deep learning neural networks (e.g., 302) on the medical image, a failure classification label (e.g., 304) that indicates that the medical imaging scanner is afflicted by a first defined scanning failure from a plurality of defined scanning failures (e.g., 402).

In various instances, act 1806 can include transmitting, by the device (e.g., via 122), an electronic notification (e.g., 1002) to a computing device associated with a technician of the medical imaging scanner, wherein the electronic notification requests that the medical imaging scanner be serviced to remedy the first defined scanning failure.

Although not explicitly shown in FIG. 18, the computer-implemented method 1800 can comprise: generating, by the device (e.g., via 118) and via execution of at least one of the one or more deep learning neural networks (e.g., 502) on the medical image and on the failure classification label, a root cause classification label (e.g., 504) that indicates that a first defined hardware component, from a plurality of defined hardware components (e.g., 202) that make up the medical imaging scanner, is malfunctioning and thereby causing the first defined scanning failure, wherein the electronic notification can indicate that the first defined scanning failure is curable by repairing or replacing the first defined hardware component.

Although not explicitly shown in FIG. 18, the computer-implemented method 1800 can comprise: estimating, by the device (e.g., via 120) and via execution of at least one of the one or more deep learning neural networks (e.g., 702) on the medical image, on the failure classification label, and on the root cause classification label, a first remaining useful life (e.g., 704) for the first defined hardware component, wherein the electronic notification can include the first remaining useful life.

Although not explicitly shown in FIG. 18, the computer-implemented method 1800 can comprise: computing, by the device (e.g., via 122) and based on a current date, a future date on which the first remaining useful life of the first defined hardware component will elapse, and wherein the electronic notification can indicate that the medical imaging scanner should be serviced no later than the future date.

Although not explicitly shown in FIG. 18, the medical imaging scanner can correspond to a digital twin (e.g., 108), the digital twin can estimate a second remaining useful life (e.g., 1602) of the first defined hardware component, and the computer-implemented method 1800 can comprise: comparing, by the device (e.g., via 122), the first remaining useful life to the second remaining useful life; estimating, by the device (e.g., via 122), in response to a determination that the second remaining useful life is not within a threshold margin of the first remaining useful life, and via execution of at least one of the one or more deep learning neural networks (e.g., 1606) on the medical image and on a current parametric value (e.g., 1604) of the digital twin, an updated parametric value (e.g., 1608) of the digital twin; and synchronizing, by the device (e.g., via 122), the digital twin to the medical imaging scanner using the updated parametric value.

Although not explicitly shown in FIG. 18, the computer-implemented method 1800 can comprise: identifying, by the device (e.g., via 122) and from a technical document repository (e.g., 1302), one or more technical documents (e.g., 1308) that are semantically relevant to the failure classification label, to the root cause classification label, and to the first remaining useful life; and synthesizing, by the device (e.g., via 122) and via execution of a large language model (e.g., 1202) on the failure classification label, on the root cause classification label, on the first remaining useful life, on the one or more technical documents, and on a tutorial prompt (e.g., 1402), a textual tutorial (e.g., 1102) explaining how to repair or replace the first defined hardware component so as to remedy the first defined scanning failure, wherein the electronic notification includes the textual tutorial.

Various embodiments described herein can be considered as a clever or inventive pipeline that utilizes deep learning so as to predict failures or malfunctions of medical imaging scanners, even in the absence of internal operability sensors. Such embodiments are inherently computer-centric and help to reduce or avoid unwanted scanner downtime, which can improve clinical outcomes for real-world medical patients. Such embodiments thus qualify as concrete technical improvements in the field of medical imaging scanners and certainly constitute a practical application of computer technology.

In various instances, machine learning algorithms or models can be implemented in any suitable way to facilitate any suitable aspects described herein. To facilitate some of the above-described machine learning aspects of various embodiments, consider the following discussion of artificial intelligence (AI). Various embodiments described herein can employ artificial intelligence to facilitate automating one or more features or functionalities. The components can employ various AI-based schemes for carrying out various embodiments/examples disclosed herein. In order to provide for or aid in the numerous determinations (e.g., determine, ascertain, infer, calculate, predict, prognose, estimate, derive, forecast, detect, compute) described herein, components described herein can examine the entirety or a subset of the data to which it is granted access and can provide for reasoning about or determine states of the system or environment from a set of observations as captured via events or data. Determinations can be employed to identify a specific context or action, or can generate a probability distribution over states, for example. The determinations can be probabilistic; that is, the computation of a probability distribution over states of interest based on a consideration of data and events. Determinations can also refer to techniques employed for composing higher-level events from a set of events or data.

Such determinations can result in the construction of new events or actions from a set of observed events or stored event data, whether or not the events are correlated in close temporal proximity, and whether the events and data come from one or several event and data sources. Components disclosed herein can employ various classification (explicitly trained (e.g., via training data) as well as implicitly trained (e.g., via observing behavior, preferences, historical information, receiving extrinsic information, and so on)) schemes or systems (e.g., support vector machines, neural networks, expert systems, Bayesian belief networks, fuzzy logic, data fusion engines, and so on) in connection with performing automatic or determined action in connection with the claimed subject matter. Thus, classification schemes or systems can be used to automatically learn and perform a number of functions, actions, or determinations.

A classifier can map an input attribute vector, z = (z₁, z₂, z₃, z₄, *zₙ*), to a confidence that the input belongs to a class, as by f(z) *= confidence*(*class*). Such classification can employ a probabilistic or statistical-based analysis (e.g., factoring into the analysis utilities and costs) to determinate an action to be automatically performed. A support vector machine (SVM) can be an example of a classifier that can be employed. The SVM operates by finding a hyper-surface in the space of possible inputs, where the hyper-surface attempts to split the triggering criteria from the non-triggering events. Intuitively, this makes the classification correct for testing data that is near, but not identical to training data. Other directed and undirected model classification approaches include, e.g., naive Bayes, Bayesian networks, decision trees, neural networks, fuzzy logic models, or probabilistic classification models providing different patterns of independence, any of which can be employed. Classification as used herein also is inclusive of statistical regression that is utilized to develop models of priority.

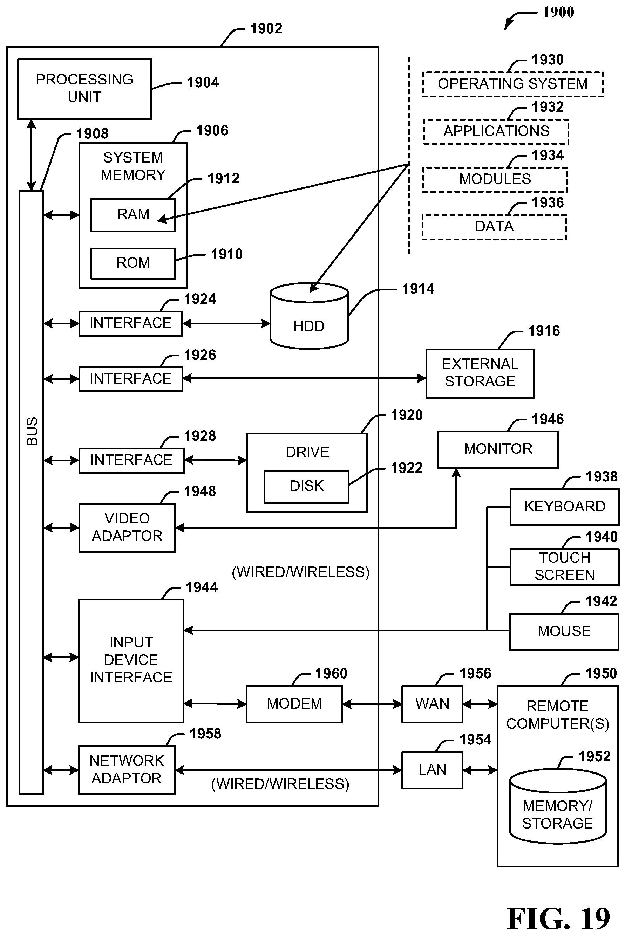

In order to provide additional context for various embodiments described herein, FIG. 19 and the following discussion are intended to provide a brief, general description of a suitable computing environment 1900 in which the various embodiments of the embodiment described herein can be implemented. While the embodiments have been described above in the general context of computer-executable instructions that can run on one or more computers, those skilled in the art will recognize that the embodiments can be also implemented in combination with other program modules or as a combination of hardware and software.

Generally, program modules include routines, programs, components, data structures, etc., that perform particular tasks or implement particular abstract data types. Moreover, those skilled in the art will appreciate that the inventive methods can be practiced with other computer system configurations, including single-processor or multi-processor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments of the embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible or non-transitory media which can be used to store desired information. In this regard, the terms "tangible" or "non-transitory" herein as applied to storage, memory or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared and other wireless media.

With reference again to FIG. 19, the example environment 1900 for implementing various embodiments of the aspects described herein includes a computer 1902, the computer 1902 including a processing unit 1904, a system memory 1906 and a system bus 1908. The system bus 1908 couples system components including, but not limited to, the system memory 1906 to the processing unit 1904. The processing unit 1904 can be any of various commercially available processors. Dual microprocessors and other multi-processor architectures can also be employed as the processing unit 1904.

The system bus 1908 can be any of several types of bus structure that can further interconnect to a memory bus (with or without a memory controller), a peripheral bus, and a local bus using any of a variety of commercially available bus architectures. The system memory 1906 includes ROM 1910 and RAM 1912. A basic input/output system (BIOS) can be stored in a non-volatile memory such as ROM, erasable programmable read only memory (EPROM), EEPROM, which BIOS contains the basic routines that help to transfer information between elements within the computer 1902, such as during startup. The RAM 1912 can also include a high-speed RAM such as static RAM for caching data.

The computer 1902 further includes an internal hard disk drive (HDD) 1914 (e.g., EIDE, SATA), one or more external storage devices 1916 (e.g., a magnetic floppy disk drive (FDD) 1916, a memory stick or flash drive reader, a memory card reader, etc.) and a drive 1920, e.g., such as a solid state drive, an optical disk drive, which can read or write from a disk 1922, such as a CD-ROM disc, a DVD, a BD, etc. Alternatively, where a solid state drive is involved, disk 1922 would not be included, unless separate. While the internal HDD 1914 is illustrated as located within the computer 1902, the internal HDD 1914 can also be configured for external use in a suitable chassis (not shown). Additionally, while not shown in environment 1900, a solid state drive (SSD) could be used in addition to, or in place of, an HDD 1914. The HDD 1914, external storage device(s) 1916 and drive 1920 can be connected to the system bus 1908 by an HDD interface 1924, an external storage interface 1926 and a drive interface 1928, respectively. The interface 1924 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 1902, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 1912, including an operating system 1930, one or more application programs 1932, other program modules 1934 and program data 1936. All or portions of the operating system, applications, modules, or data can also be cached in the RAM 1912. The systems and methods described herein can be implemented utilizing various commercially available operating systems or combinations of operating systems.

Computer 1902 can optionally comprise emulation technologies. For example, a hypervisor (not shown) or other intermediary can emulate a hardware environment for operating system 1930, and the emulated hardware can optionally be different from the hardware illustrated in FIG. 19. In such an embodiment, operating system 1930 can comprise one virtual machine (VM) of multiple VMs hosted at computer 1902. Furthermore, operating system 1930 can provide runtime environments, such as the Java runtime environment or the .NET framework, for applications 1932. Runtime environments are consistent execution environments that allow applications 1932 to run on any operating system that includes the runtime environment. Similarly, operating system 1930 can support containers, and applications 1932 can be in the form of containers, which are lightweight, standalone, executable packages of software that include, e.g., code, runtime, system tools, system libraries and settings for an application.

Further, computer 1902 can be enable with a security module, such as a trusted processing module (TPM). For instance with a TPM, boot components hash next in time boot components, and wait for a match of results to secured values, before loading a next boot component. This process can take place at any layer in the code execution stack of computer 1902, e.g., applied at the application execution level or at the operating system (OS) kernel level, thereby enabling security at any level of code execution.

A user can enter commands and information into the computer 1902 through one or more wired/wireless input devices, e.g., a keyboard 1938, a touch screen 1940, and a pointing device, such as a mouse 1942. Other input devices (not shown) can include a microphone, an infrared (IR) remote control, a radio frequency (RF) remote control, or other remote control, a joystick, a virtual reality controller or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 1904 through an input device interface 1944 that can be coupled to the system bus 1908, but can be connected by other interfaces, such as a parallel port, an IEEE 1394 serial port, a game port, a USB port, an IR interface, a BLUETOOTH^{®} interface, etc.

A monitor 1946 or other type of display device can be also connected to the system bus 1908 via an interface, such as a video adapter 1948. In addition to the monitor 1946, a computer typically includes other peripheral output devices (not shown), such as speakers, printers, etc.

The computer 1902 can operate in a networked environment using logical connections via wired or wireless communications to one or more remote computers, such as a remote computer(s) 1950. The remote computer(s) 1950 can be a workstation, a server computer, a router, a personal computer, portable computer, microprocessor-based entertainment appliance, a peer device or other common network node, and typically includes many or all of the elements described relative to the computer 1902, although, for purposes of brevity, only a memory/storage device 1952 is illustrated. The logical connections depicted include wired/wireless connectivity to a local area network (LAN) 1954 or larger networks, e.g., a wide area network (WAN) 1956. Such LAN and WAN networking environments are commonplace in offices and companies, and facilitate enterprise-wide computer networks, such as intranets, all of which can connect to a global communications network, e.g., the Internet.

When used in a LAN networking environment, the computer 1902 can be connected to the local network 1954 through a wired or wireless communication network interface or adapter 1958. The adapter 1958 can facilitate wired or wireless communication to the LAN 1954, which can also include a wireless access point (AP) disposed thereon for communicating with the adapter 1958 in a wireless mode.

When used in a WAN networking environment, the computer 1902 can include a modem 1960 or can be connected to a communications server on the WAN 1956 via other means for establishing communications over the WAN 1956, such as by way of the Internet. The modem 1960, which can be internal or external and a wired or wireless device, can be connected to the system bus 1908 via the input device interface 1944. In a networked environment, program modules depicted relative to the computer 1902 or portions thereof, can be stored in the remote memory/storage device 1952. It will be appreciated that the network connections shown are example and other means of establishing a communications link between the computers can be used.

When used in either a LAN or WAN networking environment, the computer 1902 can access cloud storage systems or other network-based storage systems in addition to, or in place of, external storage devices 1916 as described above, such as but not limited to a network virtual machine providing one or more aspects of storage or processing of information. Generally, a connection between the computer 1902 and a cloud storage system can be established over a LAN 1954 or WAN 1956 e.g., by the adapter 1958 or modem 1960, respectively. Upon connecting the computer 1902 to an associated cloud storage system, the external storage interface 1926 can, with the aid of the adapter 1958 or modem 1960, manage storage provided by the cloud storage system as it would other types of external storage. For instance, the external storage interface 1926 can be configured to provide access to cloud storage sources as if those sources were physically connected to the computer 1902.

The computer 1902 can be operable to communicate with any wireless devices or entities operatively disposed in wireless communication, e.g., a printer, scanner, desktop or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH^{®} wireless technologies. Thus, the communication can be a predefined structure as with a conventional network or simply an ad hoc communication between at least two devices.

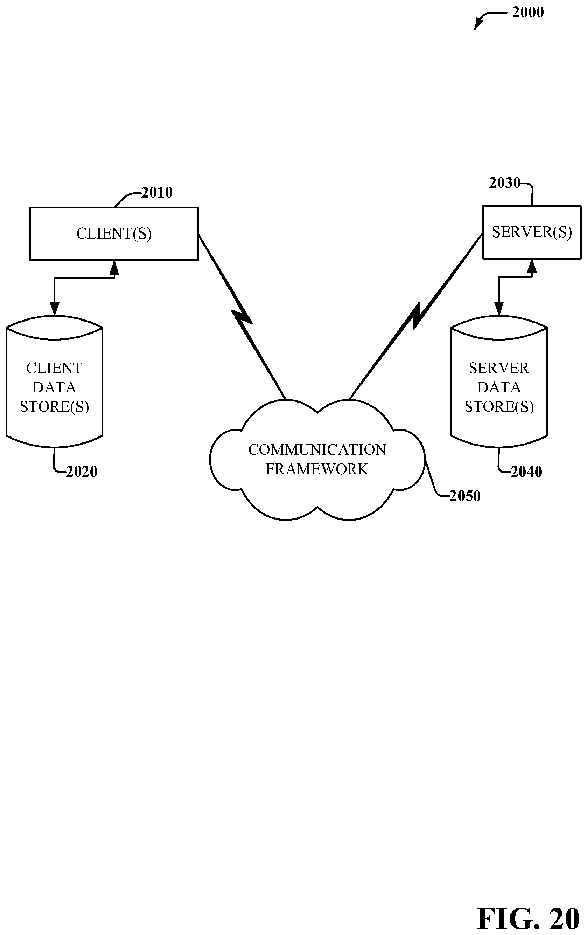

FIG. 20 is a schematic block diagram of a sample computing environment 2000 with which the disclosed subject matter can interact. The sample computing environment 2000 includes one or more client(s) 2010. The client(s) 2010 can be hardware or software (e.g., threads, processes, computing devices). The sample computing environment 2000 also includes one or more server(s) 2030. The server(s) 2030 can also be hardware or software (e.g., threads, processes, computing devices). The servers 2030 can house threads to perform transformations by employing one or more embodiments as described herein, for example. One possible communication between a client 2010 and a server 2030 can be in the form of a data packet adapted to be transmitted between two or more computer processes. The sample computing environment 2000 includes a communication framework 2050 that can be employed to facilitate communications between the client(s) 2010 and the server(s) 2030. The client(s) 2010 are operably connected to one or more client data store(s) 2020 that can be employed to store information local to the client(s) 2010. Similarly, the server(s) 2030 are operably connected to one or more server data store(s) 2040 that can be employed to store information local to the servers 2030.

Various embodiments may be a system, a method, an apparatus or a computer program product at any possible technical detail level of integration. The computer program product can include a computer readable storage medium (or media) having computer readable program instructions thereon for causing a processor to carry out aspects of various embodiments. The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium can also include the following: a portable computer diskette, a hard disk, a solid state drive such as M.2 (including non-volatile memory express (NVMe) or serial advanced technology attachment (SATA)), a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network or a wireless network. The network can comprise copper transmission cables, optical transmission fibers, wireless transmission, routers, firewalls, switches, gateway computers or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of various embodiments can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions can execute entirely on the user's computer, partly on the user's computer, as a standalone software package, partly on the user's computer and partly on a remote computer or entirely on the remote computer or server. In the latter scenario, the remote computer can be connected to the user's computer through any type of network, including a local area network (LAN) or a wide area network (WAN), or the connection can be made to an external computer (for example, through the Internet using an Internet Service Provider). In some embodiments, electronic circuitry including, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform various aspects.

Various aspects are described herein with reference to flowchart illustrations or block diagrams of methods, apparatus (systems), and computer program products according to various embodiments. It will be understood that each block of the flowchart illustrations or block diagrams, and combinations of blocks in the flowchart illustrations or block diagrams, can be implemented by computer readable program instructions. These computer readable program instructions can be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart or block diagram block or blocks. These computer readable program instructions can also be stored in a computer readable storage medium that can direct a computer, a programmable data processing apparatus, or other devices to function in a particular manner, such that the computer readable storage medium having instructions stored therein comprises an article of manufacture including instructions which implement aspects of the function/act specified in the flowchart or block diagram block or blocks. The computer readable program instructions can also be loaded onto a computer, other programmable data processing apparatus, or other device to cause a series of operational acts to be performed on the computer, other programmable apparatus or other device to produce a computer implemented process, such that the instructions which execute on the computer, other programmable apparatus, or other device implement the functions/acts specified in the flowchart or block diagram block or blocks.

The flowcharts and block diagrams in the Figures illustrate the architecture, functionality, and operation of possible implementations of systems, methods, and computer program products according to various embodiments. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams or flowchart illustration, and combinations of blocks in the block diagrams or flowchart illustration, can be implemented by special purpose hardware-based systems that perform the specified functions or acts or carry out combinations of special purpose hardware and computer instructions.

While the subject matter has been described above in the general context of computer-executable instructions of a computer program product that runs on a computer or computers, those skilled in the art will recognize that this disclosure also can or can be implemented in combination with other program modules. Generally, program modules include routines, programs, components, data structures, etc. that perform particular tasks or implement particular abstract data types. Moreover, those skilled in the art will appreciate that various aspects can be practiced with other computer system configurations, including single-processor or multiprocessor computer systems, mini-computing devices, mainframe computers, as well as computers, hand-held computing devices (e.g., PDA, phone), microprocessor-based or programmable consumer or industrial electronics, and the like. The illustrated aspects can also be practiced in distributed computing environments in which tasks are performed by remote processing devices that are linked through a communications network. However, some, if not all aspects of this disclosure can be practiced on stand-alone computers. In a distributed computing environment, program modules can be located in both local and remote memory storage devices.

As used in this application, the terms "component," "system," "platform," "interface," and the like, can refer to or can include a computer-related entity or an entity related to an operational machine with one or more specific functionalities. The entities disclosed herein can be either hardware, a combination of hardware and software, software, or software in execution. For example, a component can be, but is not limited to being, a process running on a processor, a processor, an object, an executable, a thread of execution, a program, or a computer. By way of illustration, both an application running on a server and the server can be a component. One or more components can reside within a process or thread of execution and a component can be localized on one computer or distributed between two or more computers. In another example, respective components can execute from various computer readable media having various data structures stored thereon. The components can communicate via local or remote processes such as in accordance with a signal having one or more data packets (e.g., data from one component interacting with another component in a local system, distributed system, or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. As used herein, the term "and/or" is intended to have the same meaning as "or." Moreover, articles "a" and "an" as used in the subject specification and annexed drawings should generally be construed to mean "one or more" unless specified otherwise or clear from context to be directed to a singular form. As used herein, the terms "example" or "exemplary" are utilized to mean serving as an example, instance, or illustration. For the avoidance of doubt, the subject matter disclosed herein is not limited by such examples. In addition, any aspect or design described herein as an "example" or "exemplary" is not necessarily to be construed as preferred or advantageous over other aspects or designs, nor is it meant to preclude equivalent exemplary structures and techniques known to those of ordinary skill in the art.

The herein disclosure describes non-limiting examples. For ease of description or explanation, various portions of the herein disclosure utilize the term "each," "every," or "all" when discussing various examples. Such usages of the term "each," "every," or "all" are non-limiting. In other words, when the herein disclosure provides a description that is applied to "each," "every," or "all" of some particular object or component, it should be understood that this is a non-limiting example, and it should be further understood that, in various other examples, it can be the case that such description applies to fewer than "each," "every," or "all" of that particular object or component.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor can also be implemented as a combination of computing processing units. In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory or memory components described herein can be either volatile memory or nonvolatile memory, or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or computer-implemented methods herein are intended to include, without being limited to including, these and any other suitable types of memory.

What has been described above include mere examples of systems and computer-implemented methods. It is, of course, not possible to describe every conceivable combination of components or computer-implemented methods for purposes of describing this disclosure, but many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

The descriptions of the various embodiments have been presented for purposes of illustration, but are not intended to be exhaustive or limited to the embodiments disclosed. Many modifications and variations will be apparent without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A system, comprising:
a processor (e.g., 110) that executes computer-executable components stored in a non-transitory computer-readable memory (e.g., 112), wherein the computer-executable components comprise:
an access component (e.g., 114) that accesses a medical image (e.g., 106) captured by a medical imaging scanner (e.g., 104);
a failure component (e.g., 116) that generates, via execution of at least one of one or more deep learning neural networks (e.g., 302) on the medical image, a failure classification label (e.g., 304) that indicates that the medical imaging scanner is afflicted by a first defined scanning failure from a plurality of defined scanning failures (e.g., 402); and
an action component (e.g., 122) that transmits an electronic notification (e.g., 1002) to a computing device associated with a technician of the medical imaging scanner, wherein the electronic notification requests that the medical imaging scanner be serviced to remedy the first defined scanning failure.

2. The system of claim 1, wherein the computer-executable components further comprise:
a cause component (e.g., 118) generates, via execution of at least one of the one or more deep learning neural networks (e.g., 502) on the medical image, a root cause classification label (e.g., 504) that indicates that a first defined hardware component, from a plurality of defined hardware components (e.g., 202) that make up the medical imaging scanner, is malfunctioning and thereby causing the first defined scanning failure, wherein the electronic notification indicates that the first defined scanning failure is curable by repairing or replacing the first defined hardware component.

3. The system of claim 2, wherein the computer-executable components further comprise:
a life component (e.g., 120) that estimates, via execution of at least one of the one or more deep learning neural networks (e.g., 702) on the medical image, a first remaining useful life (e.g., 704) for the first defined hardware component, wherein the electronic notification includes the first remaining useful life.

4. The system of claim 3, wherein the action component computes, based on a current date, a future date on which the first remaining useful life of the first defined hardware component will elapse, and wherein the electronic notification indicates that the medical imaging scanner should be serviced no later than the future date.

5. The system of claim 3, wherein the medical imaging scanner corresponds to a digital twin (e.g., 108), wherein the digital twin estimates a second remaining useful life (e.g., 1602) of the first defined hardware component, and wherein the action component compares the first remaining useful life to the second remaining useful life.

6. The system of claim 5, wherein the action component estimates, in response to a determination that the second remaining useful life is not within a threshold margin of the first remaining useful life and via execution of at least one of the one or more deep learning neural networks (e.g., 1606) on the medical image and on a current parametric value (e.g., 1604) of the digital twin, an updated parametric value of the digital twin (e.g., 1608), and wherein the action component synchronizes the digital twin to the medical imaging scanner using the updated parametric value.

7. The system of claim 3, wherein the action component:
identifies, from a technical document repository (e.g., 1302), one or more technical documents (e.g., 1308) that are semantically relevant to the failure classification label, to the root cause classification label, and to the first remaining useful life; and
synthesizes, via execution of a large language model (e.g., 1202) on the failure classification label, on the root cause classification label, on the first remaining useful life, on the one or more technical documents, and on a tutorial prompt (e.g., 1402), a textual tutorial (e.g., 1102) explaining how to repair or replace the first defined hardware component so as to remedy the first defined scanning failure, wherein the electronic notification includes the textual tutorial.

8. A computer-implemented method, comprising:
accessing, by a device (e.g., via 114) operatively coupled to a processor, a medical image (e.g., 106) captured by a medical imaging scanner (e.g., 104);
generating, by the device (e.g., via 116) and via execution of at least one of one or more deep learning neural networks (e.g., 302) on the medical image, a failure classification label (e.g., 304) that indicates that the medical imaging scanner is afflicted by a first defined scanning failure from a plurality of defined scanning failures (e.g., 402); and
transmitting, by the device (e.g., via 122), an electronic notification (e.g., 1002) to a computing device associated with a technician of the medical imaging scanner, wherein the electronic notification requests that the medical imaging scanner be serviced to remedy the first defined scanning failure.

9. The computer-implemented method of claim 8, further comprising:
generating, by the device (e.g., via 118) and via execution of at least one of the one or more deep learning neural networks (e.g., 502) on the medical image, a root cause classification label (e.g., 504) that indicates that a first defined hardware component, from a plurality of defined hardware components (e.g., 202) that make up the medical imaging scanner, is malfunctioning and thereby causing the first defined scanning failure, wherein the electronic notification indicates that the first defined scanning failure is curable by repairing or replacing the first defined hardware component.

10. The computer-implemented method of claim 9, further comprising:
estimating, by the device (e.g., via 120) and via execution of at least one of the one or more deep learning neural networks (e.g., 702) on the medical image, a first remaining useful life (e.g., 704) for the first defined hardware component, wherein the electronic notification includes the first remaining useful life.

11. The computer-implemented method of claim 10, further comprising:
computing, by the device (e.g., via 122) and based on a current date, a future date on which the first remaining useful life of the first defined hardware component will elapse, and wherein the electronic notification indicates that the medical imaging scanner should be serviced no later than the future date.

12. The computer-implemented method of claim 10, wherein the medical imaging scanner corresponds to a digital twin (e.g., 108), wherein the digital twin estimates a second remaining useful life (e.g., 1602) of the first defined hardware component, and further comprising:
comparing, by the device (e.g., via 122), the first remaining useful life to the second remaining useful life.

13. The computer-implemented method of claim 12, further comprising:
estimating, by the device (e.g., via 122), in response to a determination that the second remaining useful life is not within a threshold margin of the first remaining useful life, and via execution of at least one of the one or more deep learning neural networks (e.g., 1606) on the medical image and on a current parametric value (e.g., 1604) of the digital twin, an updated parametric value (e.g., 1608) of the digital twin; and
synchronizing, by the device (e.g., via 122), the digital twin to the medical imaging scanner using the updated parametric value.

14. The computer-implemented method of claim 10, further comprising:
identifying, by the device (e.g., via 122) and from a technical document repository (e.g., 1302), one or more technical documents (e.g., 1308) that are semantically relevant to the failure classification label, to the root cause classification label, and to the first remaining useful life; and
synthesizing, by the device (e.g., via 122) and via execution of a large language model (e.g., 1202) on the failure classification label, on the root cause classification label, on the first remaining useful life, on the one or more technical documents, and on a tutorial prompt (e.g., 1402), a textual tutorial (e.g., 1102) explaining how to repair or replace the first defined hardware component so as to remedy the first defined scanning failure, wherein the electronic notification includes the textual tutorial.

15. A computer program product for facilitating scanner fault prediction via image-based deep learning, the computer program product comprising a non-transitory computer-readable memory (e.g., 112) having program instructions embodied therewith, the program instructions executable by a processor (e.g., 110) to cause the processor to:
access a medical image (e.g., 106) captured by a medical imaging scanner (e.g., 104);
generate, via execution of at least one of one or more deep learning neural networks (e.g., 302) on the medical image, a failure classification label (e.g., 304) that indicates that the medical imaging scanner is afflicted by a first defined scanning failure from a plurality of defined scanning failures (e.g., 402); and
transmit an electronic notification (e.g., 1002) to a computing device associated with a technician of the medical imaging scanner, wherein the electronic notification requests that the medical imaging scanner be serviced to remedy the first defined scanning failure.
